# EUROPEAN PATENT SPECIFICATION

(11) **EP 0 528 271 B1**
(45) Date of publication and mention of the grant of the patent: **23.04.1997**
(21) Application number: 92113322.9
(22) Date of filing: 05.08.1992
(51) Int. Cl.: C07K 14/635, C12N 15/16, C12N 15/18, C12N 1/21, C12N 5/10, C12P 21/02

(54) **Human parathyroid hormone muteins and production thereof**
Muteine des menschlichen Parathormons und ihre Herstellung
Mutéines de l'hormone parathyroidienne humaine et leur production

(30) Priority: 07.08.1991 JP 198056/91; 26.06.1992 JP 169713/92
(43) Date of publication of application: 24.02.1993
(73) Proprietor: TAKEDA CHEMICAL INDUSTRIES, LTD., Chuo-ku, Osaka 541 (JP)
(72) Inventor: Fukuda, Tsunehiko, Nishikyo-ku, Kyoto 610-11 (JP)
(74) Representative: von Kreisler, Alek, Dipl.-Chem.

(56) References cited:
- EP-A- 0 139 076
- EP-A- 0 341 963
- EP-A- 0 451 867
- WO-A-88/03165
- WO-A-90/14415

## Description

### BACKGROUND OF THE INVENTION

The present invention relates to novel human parathyroid hormone derivatives useful as hormone remedies and the production thereof.

Parathyroid hormone (hereinafter also briefly referred to as PTH) is a polypeptide hormone consisting of 84 amino acids which is secreted from the parathyroid, and one of the most important regulators for calcium metabolism. Accordingly, the application of human PTH to various bone diseases such as hypoparathyroidism and osteoporosis and further the application of human PTH antagonists to hypercalcemia and the like have been strongly desired.

The DNA sequence of human PTH was first revealed by G. N. Hendy et al. [Proc. Natl. Acad. Sci. U.S.A., 78, 7365-7369 (1981)]. Since then, many attempts have been made to obtain human PTH by genetic engineering techniques. Recently, it has been expressed in amounts satisfiable from the industrial viewpoint with much effort [for example, Wing L. Sung et al., J. Biol. Chem., 266, 2831-2835 (1991) and EP-A-0 483 509].

Human PTH has the following amino acid sequence: 
Ser-Val-Ser-Glu-Ile-Gln-Leu-Met-His-Asn-Leu-Gly-Lys-
1               5                   10
His-Leu-Asn-Ser-Met-Glu-Arg-Val-Glu-Trp-Leu-Arg-Lys-Lys-
15                  20                  25
Leu-Gln-Asp-Val-His-Asn-Phe-Val-Ala-Leu-Gly-Ala-Pro-Leu-
        30                  35                  40
 
Ala-Pro-Arg-Asp-Ala-Gly-Ser-Gln-Arg-Pro-Arg-Lys-Lys-Glu-
45 50 55
Asp-Asn-Val-Leu-Val-Glu-Ser-His-Glu-Lys-Ser-Leu-Gly-Glu-
60 65
Ala-Asp-Lys-Ala-Asp-Val-Asn-Val-Leu-Thr-Lys-Ala-Lys-Ser-Gln
70 75 80
 Its biological activity has previously been known to be reproducible by the fragment consisting of amino acid residues situated in the 1- to 34-positions on the N-terminal side (the positions of amino acid residues are hereinafter represented by the numbers corresponding to those of the sequence of human PTH (1-84) taking Ser as the 1-position) [G. W. Tregear et al., Endocrinology, 93, 1349-1353 (1973)], and many derivatives thereof have been synthesized. As to this (1-34) fragment, a peptide in which 2 to 6 amino acid residues on the N-terminal side are deleted is known to have PTH antagonist activity. Furthermore, the binding activity of a C-terminal portion, from the 35-position on, to a receptor [L. G. Rao et al., Endocrinology, 117, 1632-1636 (1985)] and the activating action thereof to alkaline phosphatase [T. M. Murray et al., Endocrinology, 124 1097-1099 (1989)] have recently been disclosed.

However, when natural type PTH(1-84) is actually used as a drug, it has some problems to be solved. For example, Met residues in a peptide chain are gradually oxidized even under ordinary conditions, and PTH whose Met residue is oxidized is significantly reduced in biological activity [A. L. Frelinger III and J. E. Zull, J. Biol. Chem., 259, 5507-5513 (1984)]. Furthermore, it is generally preferred to use drugs as non-injection drugs from the simplicity and easiness of their administration. The modification of human PTH (1-84) is considered to make it possible to change the physicochemical properties of the drugs, for example, to allow the drugs to be easily absorbed from the mucous membrane.

It is the most common method to replace an amino acid(s) in a peptide chain by another amino acid(s) to attempt to improve the biological and physicochemical properties of a biologically active peptide, in order to solve such problems. Previously, the present inventors provided a high expression system of human PTH (1-84) in Escherichia coli (EP-A 0 483 509), and succeeded in obtaining anti-oxidative derivatives by substituting other amino acid for Met residues of human PTH, utilizing this expression system. In addition, the present inventors obtained derivatives in which various amino acid residues in the center portion of the peptide chain are substituted by Cys residues. For example, a highly lipophilic group is specifically introduced into this SH group, or a dimer is formed through an S-S bond, whereby the derivative can be derived to an agonist having high affinity to a receptor or difficulty to undergo decomposition in vivo. Cyano-group can be introduced into the side-chain of the Cys residue followed by cleavage of the peptide bond to give an active fragment of PTH. Further, compounds in which several amino acid residues on the N-terminal side of PTH (1-34) are deleted are known to function as inhibitors [N. Horiuchi et al., Science, 220, 1053-1055 (1983)].

### SUMMARY OF THE INVENTION

The present invention provides antagonists in which several amino acid residues on the N-terminal side of human PTH containing the C-terminal peptide chain are deleted, and peptides obtained by further subjecting the antagonists to the amino acid substitution mentioned above. These compounds have more desirable properties in clinical application.

In accordance with the present invention, there are provide (1) a human parathyroid hormone mutein comprising at least one modification which is selected from the group consisting of (i) deletion of 3 to 6 amino acid residues on the N-terminal side in the amino acid sequence of human parathyroid hormones, (ii) substitution of another lipophilic amino acid residue for at least one methionine residue in said amino acid sequence, and (iii) substitution of a cysteine residue for one amino acid residue within the region of amino acid residue Nos. 34 to 47 in said amino acid sequence provided that a deletion according to (i) is accompanied by a substitution according to (ii) and/or (iii); (2) a recombinant DNA having a nucleotide sequence coding for the human parathyroid hormone mutein described in (1); (3) a vector containing the recombinant DNA described in (2); (4) a vector in which the recombinant DNA described in (2) is inserted into the region controlled by an E. coli T7 promoter; (5) a transformant which is transformed by the recombinant DNA described in (2); and (6) a process for producing a human parathyroid hormone mutein which comprises cultivating the transformant described in (5) in a culture medium.

### BRIEF DESCRIPTION OF THE DRAWINGS

Fig. 1 shows DNA sequences and amino acid sequences of the present invention corresponding to human PTH and human PTH analogues lacking its N-terminal portion;
Fig. 2 shows examples of cleavage of DNA fragments in synthesizing genes coding for human PTH of the present invention and analogues lacking its N-terminal portion;
Fig. 3 shows examples of DNA fragments for producing a synthetic gene corresponding to a human PTH analogue of the present invention;
Fig. 4 is a schematic showing the production of a synthetic gene coding for a human PTH analogue by binding the respective DNA fragments shown in Fig. 3;
Fig. 5 shows the construction scheme for an expression plasmid which contains the synthetic gene shown in Fig. 4 is incorporated, taking human PTH (5-84) (Reference Example 7) as an example;
Fig. 6 shows a DNA sequence and an amino acid sequence corresponding to human PTH (5-84) of Reference Example 7 ;
Fig. 7 shows the construction scheme for plasmid pU-C35PTH obtained in Example 1;
Fig. 8 is a representation shows the construction scheme for plasmid pE-C35PTH obtained in Example 1;
Fig. 9 shows a DNA sequence and an amino acid sequence corresponding to [cys³⁵] human PTH obtained in Example 1;
Fig. 10 is a representation showing the construction scheme for plasmid pU-L18PTH obtained in Example 2;

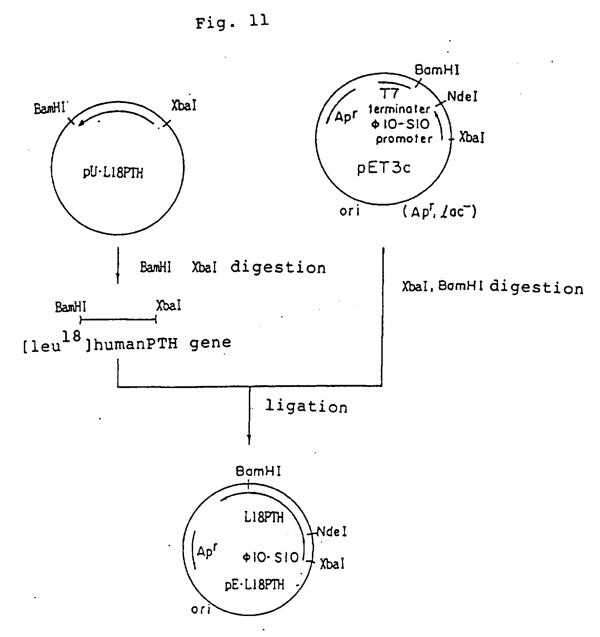

Fig. 11 is a representation showing the construction scheme for plasmid pE-L18PTH obtained in Example 2;
Fig. 12 shows a DNA sequence and an amino acid sequence corresponding to [Leu¹⁸] human PTH in Example 2;
Fig. 13 shows results of SDS-PAGE after expression of a desired protein in Reference Example 7, together with results of control experiments, wherein lanes 1 to 5 are as follows:
   Lane 1: Molecular weight marker
   Lane 2: E. coli strain culture solution (10 µl) carrying plasmid pE-PTH (5-84) after induction of IPTG
   Lane 3: E. coli strain culture solution (10 µl) carrying plasmid pE-PTH (5-84) without induction of IPTG
   Lane 4: Human PTH expression strain culture solution (10 µl) after induction of IPTG
   Lane 5: Human PTH expression strain culture solution without induction of IPTG;

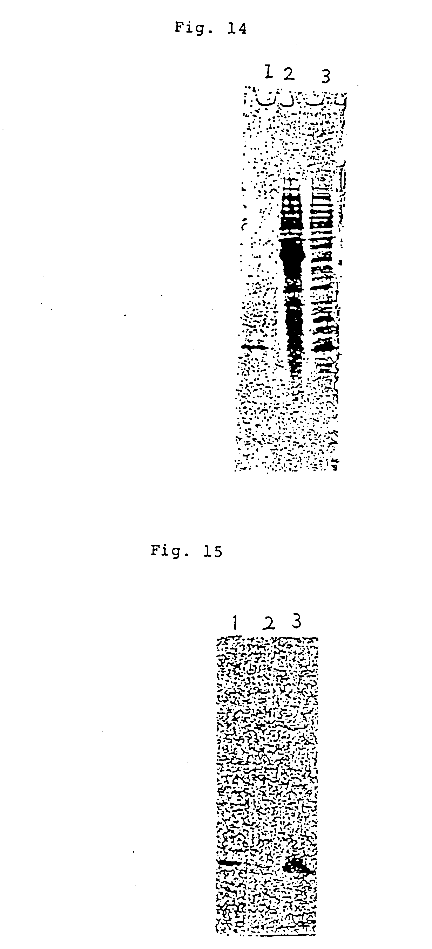

Fig. 14 shows results of SDS-PAGE after expression of a desired protein obtained in Example 1, together with results of control experiments:
   Lane 1: Human PTH (1 µg)
   Lane 2: E. coli strain culture solution (10 µl) not carrying plasmid pE-C35PTH after addition of IPTG
   Lane 3: E. coli strain culture solution (10 µl) carrying plasmid pE-C35PTH after addition of IPTG;
Fig. 15 shows a western blotting of a desired protein obtained in Example 1;

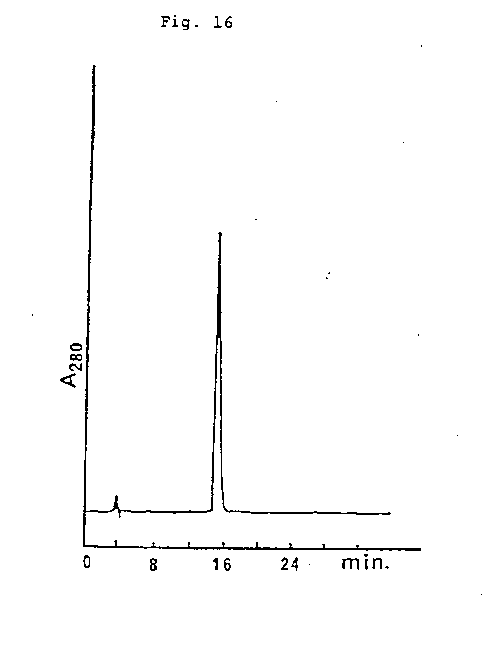

Fig. 16 shows an HPLC chromatogram of purified [Cys³⁵] human PTH obtained in Example 1, column: YMC ODS A-303 4.6x250 mm, elution conditions: a linear gradient of 0 minute (30% acetonitrile containing 0.1% TFA) → 30 minutes (38% acetonitrile containing 0.1% TFA), flow rate: 1 ml/minute;

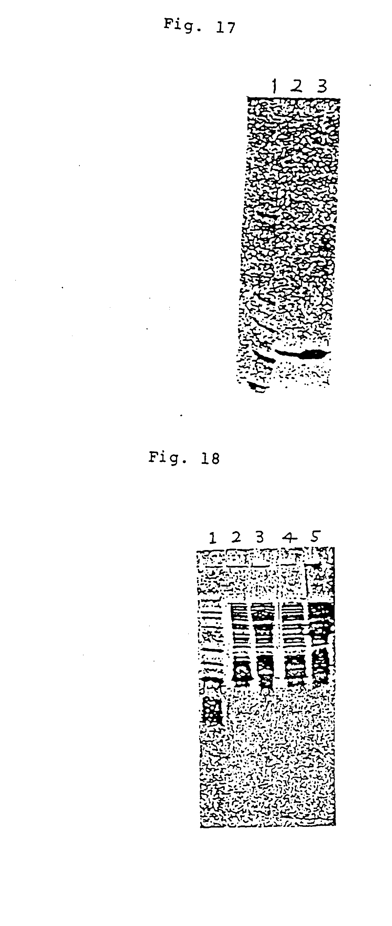

Fig. 17 shows results of SDS-PAGE (18% polyacrylamide) of purified [Cys³⁵] human PTH obtained in Example 1:
   Lane 1: Molecular weight marker
   Lane 2: Human PTH
   Lane 3: [Cys³⁵] human PTH;
Fig. 18 shows results of SDS-PAGE of expression [Leu¹⁸] human PTH obtained in Example 2:
   Lane 1: Molecular weight marker
   Lane 2: E. coli strain culture solution (10 µl) carrying plasmid pE-L18PTH after addition of IPTG
   Lane 3: E. coli strain culture solution (10 µl) carrying plasmid pE-L18PTH without addition of IPTG;

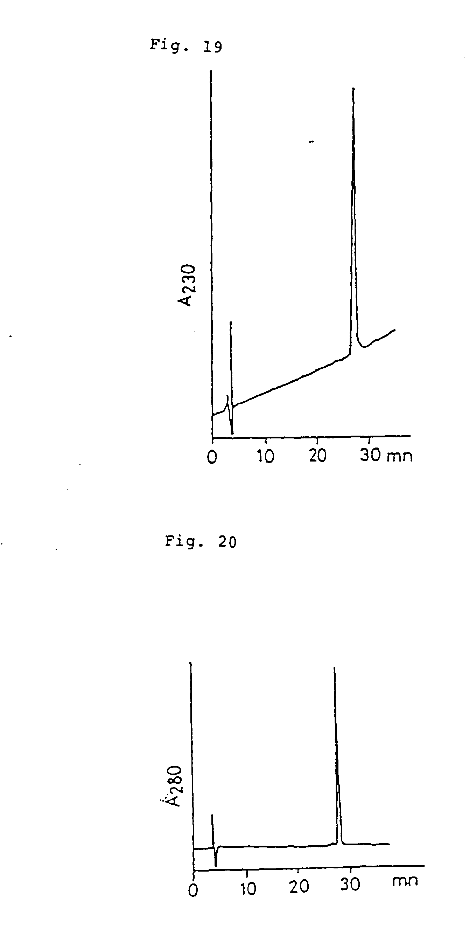

Fig. 19 shows an HPLC chromatogram of purified [Leu¹⁸] human PTH obtained in Example 2;
Fig. 20 shows an HPLC chromatogram of purified [Leu⁸] human PTH obtained in Example 3;

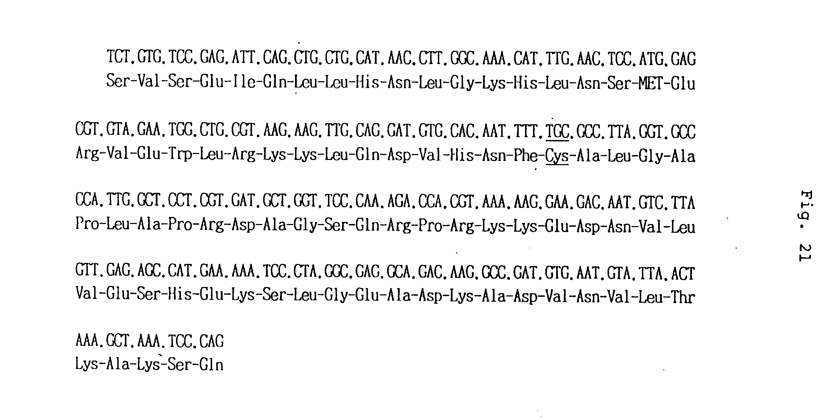

Fig. 21 shows a DNA sequence and an amino acid sequence corresponding to [Leu⁸] human PTH obtained in Example 3;

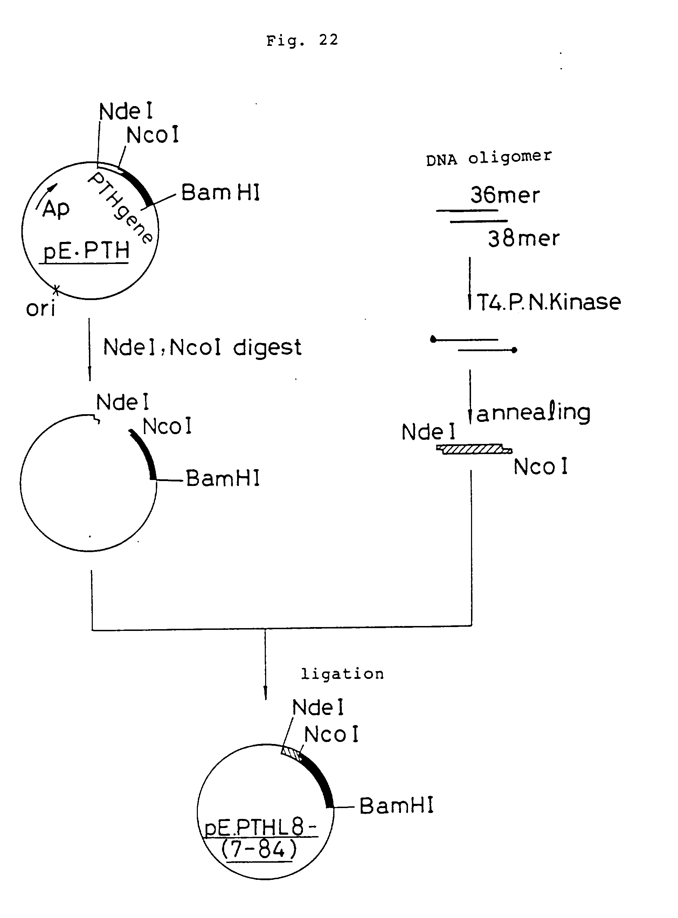

Fig. 22 shows the construction scheme for an expression plasmid of [Leu⁸] human PTH (7-84) obtained in Example 4;

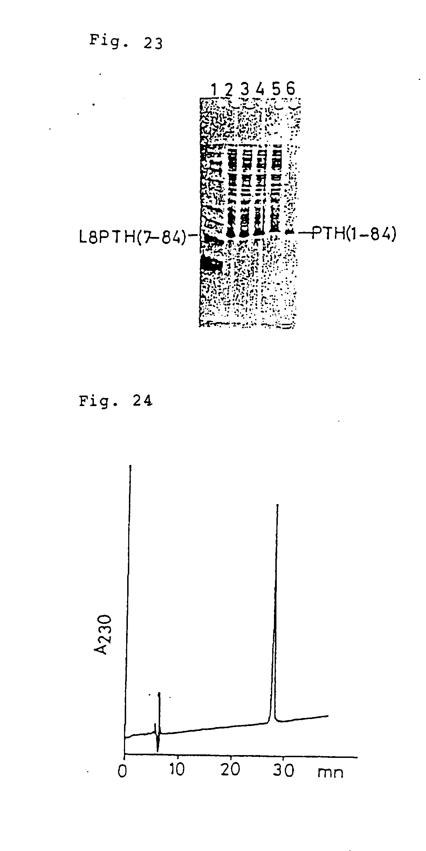

Fig. 23 shows results of SDS-PAGE after expression of a desired protein in Example 4:
   Lane 1: Molecular weight marker,
   Lanes 2 to 4: Whole cell lysate of MM294(DE3)/pE-L8PTH (7-84) induced by IPTG, in which [Leu⁸] human PTH (7-84) is expressed,
   Lane 5: Whole cell lysate in which IPTG is added to E. coli having a gene coding for human PTH to express human PTH,
   Lane 6: Standard human PTH (1-84) (1 µg)

   The gel was stained with Coomassie Blue;
Fig. 24 shows an HPLC chromatogram of purified [Leu⁸] human PTH (7-84) obtained in Example 4;

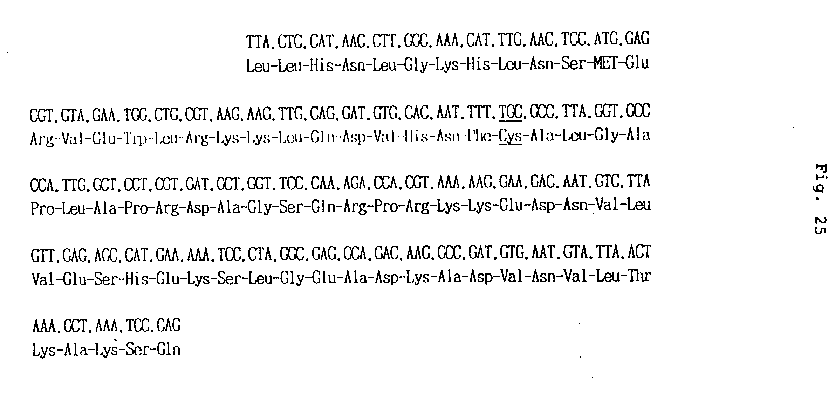

Fig. 25 shows a DNA sequence and an amino acid sequence corresponding to [Leu⁸] human PTH (7-84) obtained in Example 4;

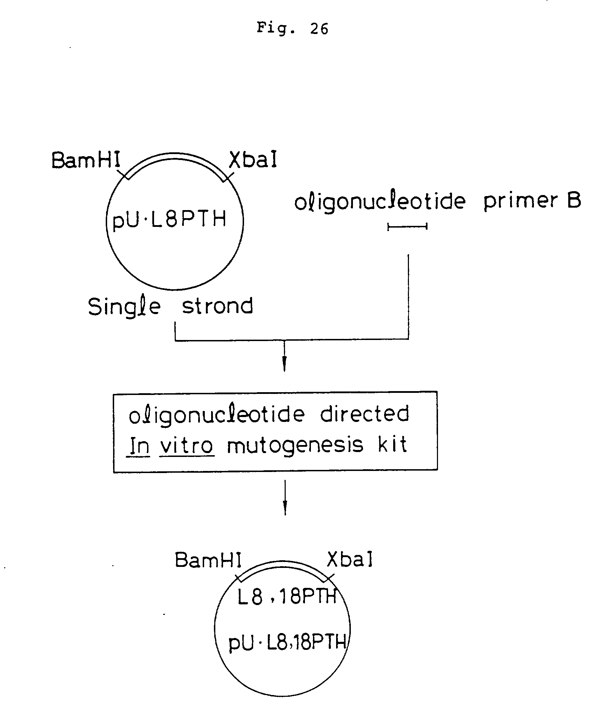

Fig. 26 shows the construction scheme for plasmid pU-L8,18PTH obtained in Example 5;

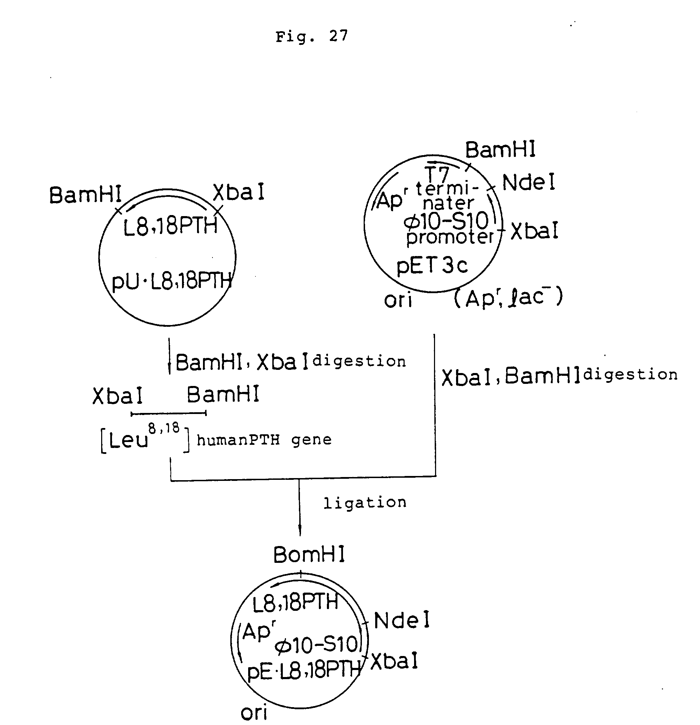

Fig. 27 shows the construction scheme for plasmid pE-L8,18PTH obtained in Example 5;

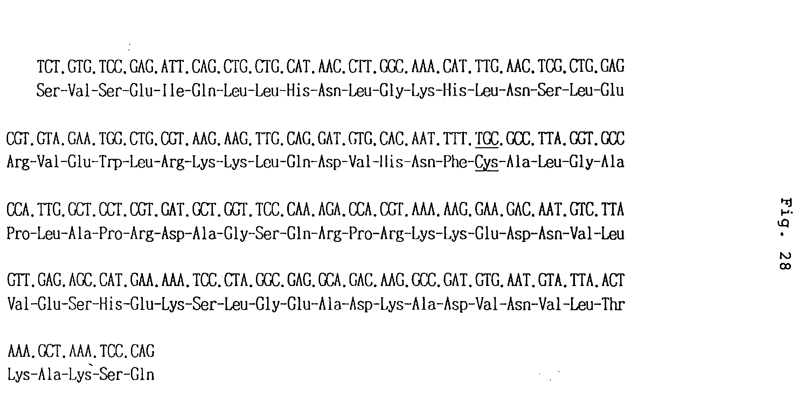

Fig. 28 shows a DNA sequence and an amino acid sequence corresponding to [Leu^{8,18}] human PTH obtained in Example 5; and

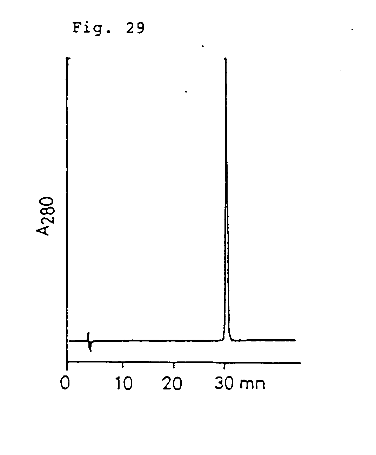

Fig. 29 shows an HPLC chromatogram of purified [Leu^{8,18}] human PTH obtained in Example 5.

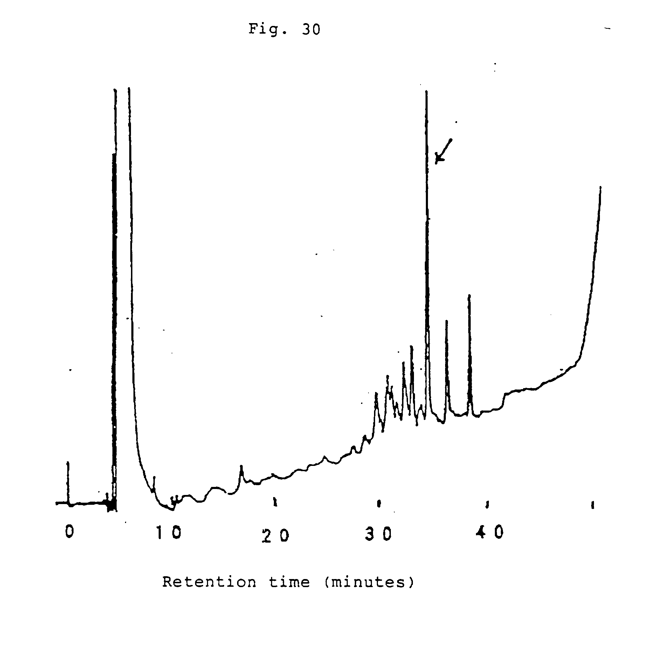

Fig. 30 shows a result of reverse phase HPLC column chromatography in Reference Example 4.

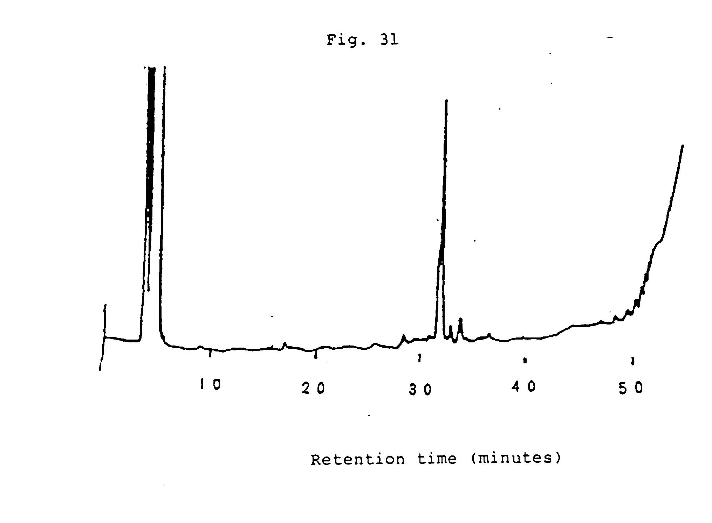

Fig. 31 shows a result of reverse phase HPLC column chromatography in Reference Example 5.

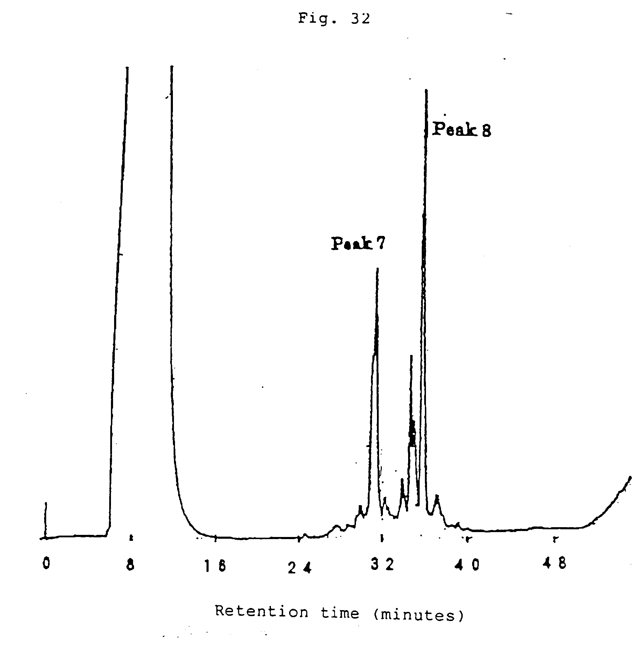

Fig. 32 shows a result of reverse phase HPLC column chromatography in Reference Example 6.

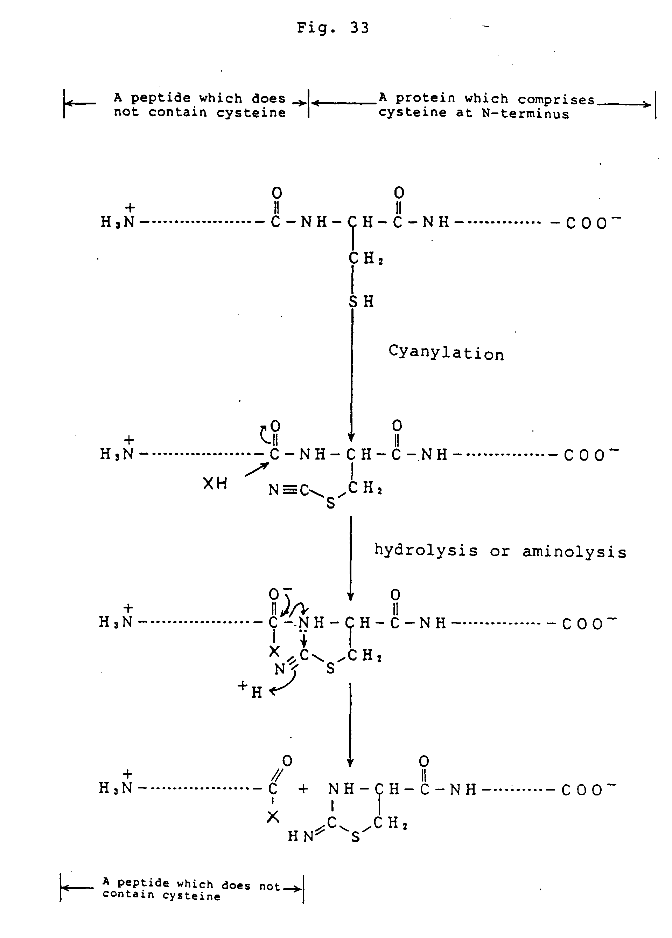

Fig. 33 shows a reaction mechanism of cleavage of human PTH.

### DESCRIPTION OF THE PREFERRED EMBODIMENTS

For the PTH muteins of the present invention, any modification may be selected depending on their purpose, as long as it is at least one selected from (i), (ii) and (iii) described above. Furthermore, theses modifications may be combined. For example, the muteins which can be preferably used according to their purpose include a mutein in which N-terminal 2 to 6 amino acid residues are deleted and methionine residues are substituted by other lipophilic amino acid residues; a mutein in which N-terminal 2 to 6 amino acid residues are deleted and one amino acid residue within the region of amino acid residue Nos. 34 to 47 is substituted by a cysteine residue; a mutein in which at least one methionine residue is substituted by other lipophilic amino acid residue and one amino acid residue within the region of amino acid residue Nos. 34 to 47 is substituted by a cysteine residue; and a mutein in which N-terminal 2 to 6 amino acid residues are deleted, at least one methionine residue is substituted by other lipophilic amino acid residue and one amino acid residue within the region of amino acid residue Nos. 34 to 47 is substituted by a cysteine residue.

In the present invention, any lipophilic amino acid residue may be used for substitution for the methionine residue situated at the 8- or 18-position, as long as it is a lipophilic amino acid residue constituent in a naturally occurring protein. Examples of such lipophilic amino acid residues include aromatic amino acid residues such as phenylalanine, tyrosine and tryptophan, and relatively long-chain aliphatic amino acid residues such as isoleucine, leucine and valine.

Examples of the human parathyroid hormone muteins of the present invention include a peptide represented by the following general formula (I) and salts thereof: 
R1-R2-His-Asn-Leu-Gly-Lys-His-Leu-Asn-Ser-R3-Glu-Arg-
Val-Glu-Trp-Leu-Arg-Lys-Lys-Leu-Gln-Asp-Val-His-Asn-R4-R5-
Ala- R6-Gly-R7-Pro-R8-Ala-R9-R10-Asp-Ala-Gly-Ser-Glu-Arg-Pro-
Arg-Lys-Lys-Glu-Asp-Asn-Val-Leu-Val-Glu-Ser-His-Glu-Lys-
Ser-Leu-Gly-Glu-Ala-Asp-Lys-Ala-Asp-Val-Asn-Val-Leu-Thr-
Lys-Ala-Lys-Ser-Gln (I)
 wherein R₁ represents Leu (SEQ ID:NO:1), Gln-Leu (SEQ ID:NO:2), Ile-Gln-Leu (SEQ ID:NO:3), Glu-Ile-Gln-Leu (SEQ ID NO:4) or Ser-Val-Ser-Glu-Ile-Gln-Leu (SEQ ID:NO:5); R₂ represents Leu, Ile, Val or Met; R₃ represents Leu, Ile, Val or Met; R₄ represents Cys or Phe; R₅ represents Cys or Val; R₆ represents Cys or Leu; R₇ represents Cys or Ala; R₈ represents Cys or Leu; R₉ represents Cys or Pro; and R₁₀ represents Cys or Arg, with the proviso that the same structure as that of human parathyroid hormone and mere deletion muteins thereof are excluded.

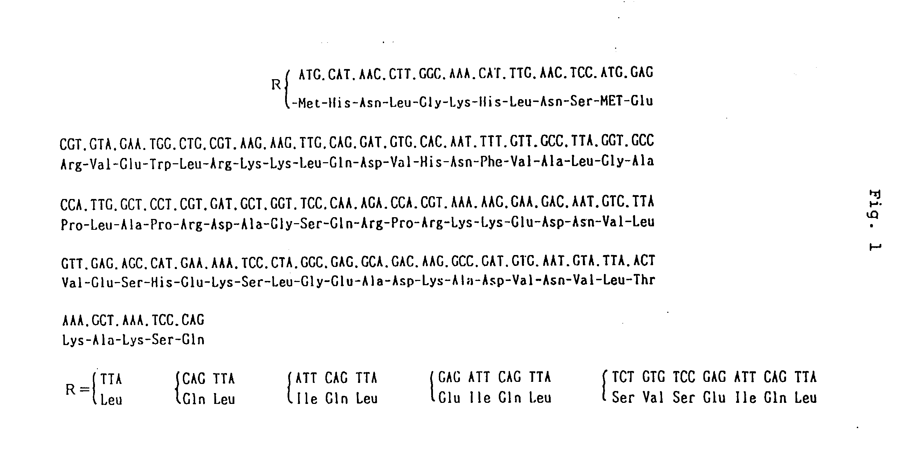

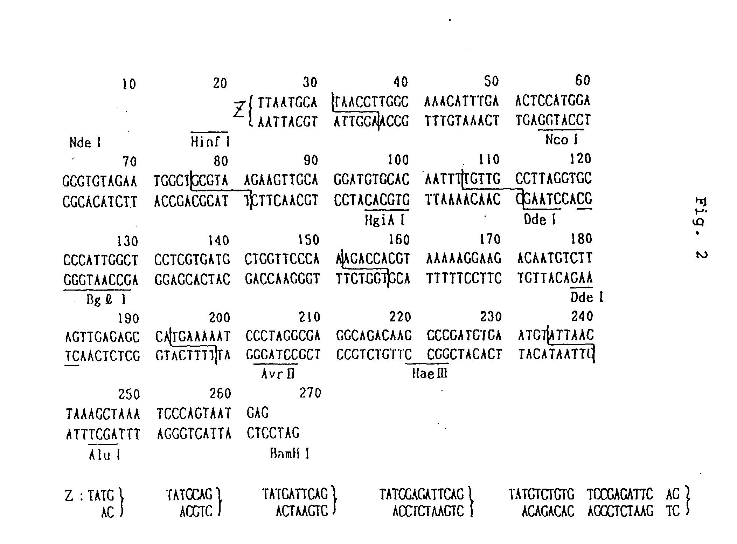

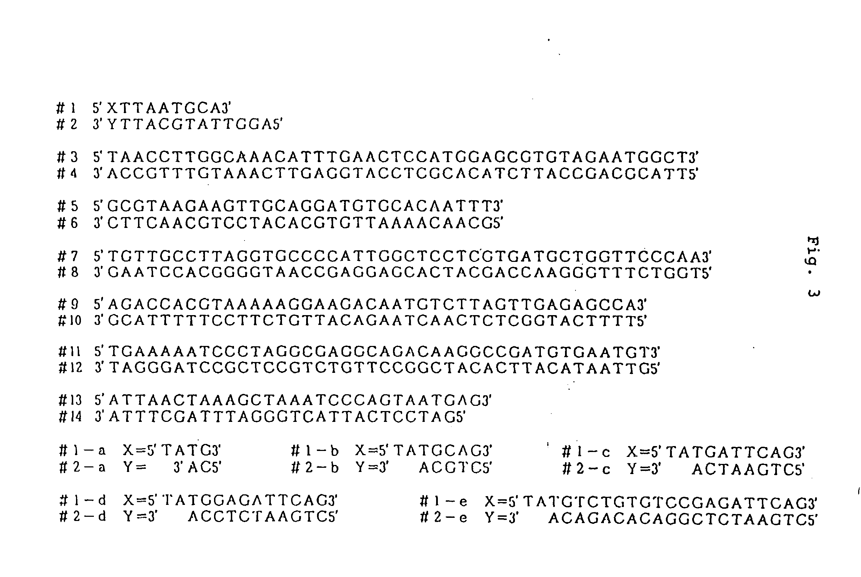

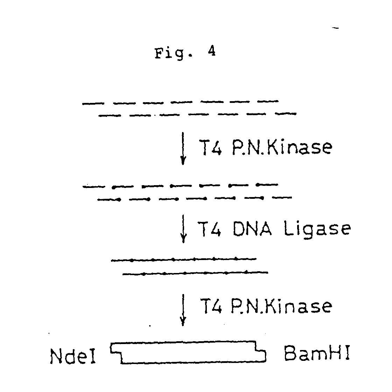

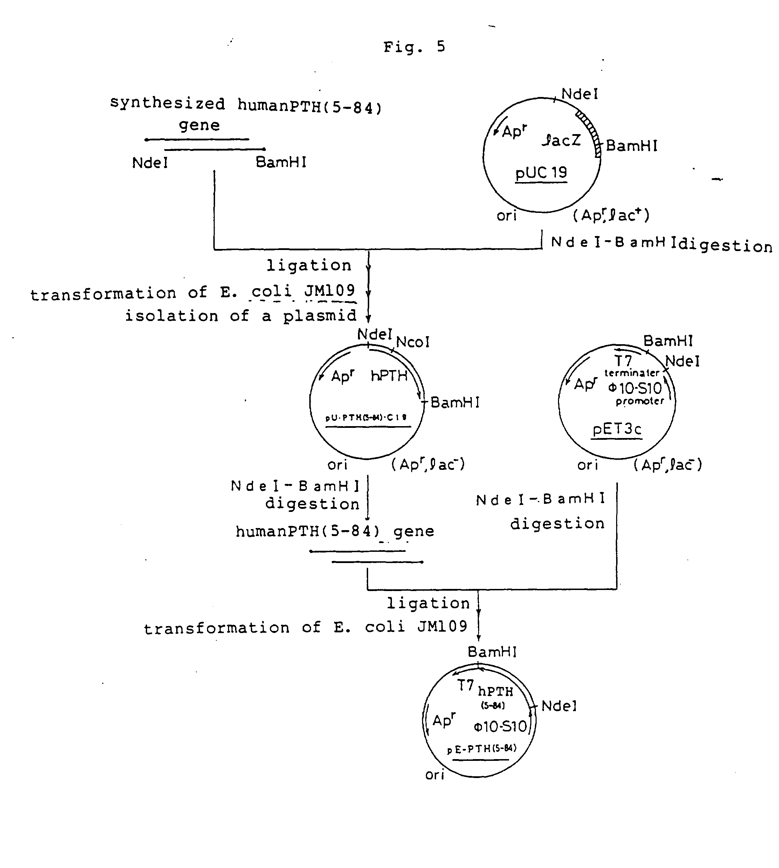

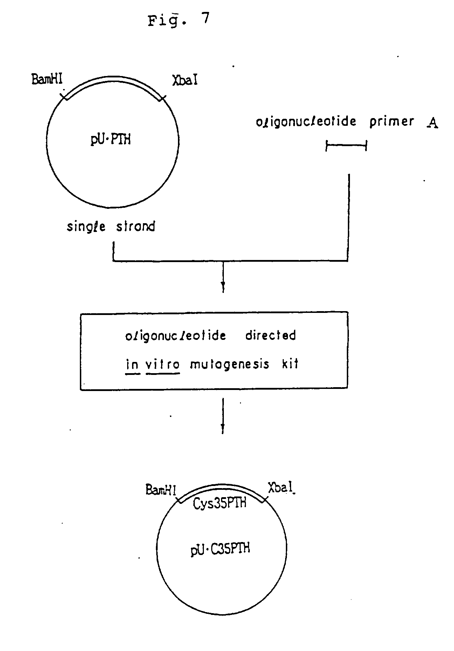

In order to produce the human PTH mutein of the present invention, a gene coding for the amino acid sequence of human PTH (1-84) (for example, EP-A-0 483 509) is converted to a gene coding for the desired mutein using a conventional DNA technique, for example, site-directed mutagenesis (for example, Fig. 7). As to the N-terminal portion lacking human PTH muteins, for example, antagonist derivatives lacking N-terminal amino acid sequences such as Ser-Val-Ser, Ser-Val-Ser-Glu, Ser-Val-Ser-Glu-Ile and Ser-Val-Ser-Glu-Ile-Gln (Fig. 1), genes of human PTH (4-84), human PTH (5-84), human PTH (6-84) and human PTH (7-84) (Fig. 2) (SEQ ID:NOs: 24 to 31) are first prepared from synthetic oligomers (Figs. 3 and 4), and inserted into vectors (Fig. 5). With respect to muteins further subjected to the amino acid substitution, the site-directed mutagenesis is applied to the corresponding genes, respectively, to obtain the desired genes. This mutagenesis, which is well known, is described in R. F. Lather and J. P. Lecoq, Genetic Engineering, Academic Press, p.31-50 (1983). Mutagenesis directed to oligonucleotide is described in M. Smith and S. Gillam, Genetic Engineering: Principles and Methods, Plenum Press, vol.3, p.1-32 (1981).

Structural genes coding for the various muteins of the present invention different in chain length subjected to the amino acid substitution are produced, for example, by the steps of:
(a) hybridizing single-stranded DNAs each of which comprises one strand of a structural gene of human PTH with mutagenic oligonucleotide primers,
(b) elongating the primers with DNA polymerase to form mutational heteroduplexes, and
(c) replicating the mutational heteroduplexes.

The size of the oligonucleotide primer depends on conditions required for stable hybridization of the primer to a gene region to which mutation is to be introduced, and on limitations in currently available methods of oligonucleotide synthesis. The factors (for example, the overall size and the size of a mismatching portion at a mutation site) to be considered in designing the oligonucleotide used for mutagenesis directed by the oligonucleotide are described by M. Smith and S. Gillam in the above literature. In general, the overall length of the oligonucleotide is such a length that stable, unique hybridization at the mutation site is optimized, and the extensions from the mutation site to the 5'- and the 3'-termini are adjusted in size so as to be sufficient to prevent mutation editing due to the exonuclease of DNA polymerase. The oligonucleotides used for mutagenesis in accordance with the present invention usually contain about 12 to 40 nucleotides, and preferably 14 to 24 nucleotides. These usually contain at least 3 nucleotides on the 3'-terminal side from the codon to be changed.

For example, for the purpose of obtaining a mutein whose constituent amino acid, valine, is substituted by cysteine, site-directed mutagenesis is conducted, using a synthetic nucleotide primer which changes a valine codon to a cysteine codon, thereby producing a modified human PTH gene.

For example, in order to change methionine at the 8-position of human PTH to leucine, the primer is hybridized with an anti-sense chain of the human PTH gene. Preferred examples of the nucleotide primers include

5′-TCCGAGATTCAGCTGCTGCATAACCTT-3′ (SEQ ID NO:6).

When the methionine is changed to isoleucine, examples of the nucleotide primers include

5'-TCCGAGATTCAGATCCTGCATAACCTT-3' (SEQ ID NO:7).

When the methionine is changed to threonine, examples of the nucleotide primers include

5'-TCCGAGATTCAGACGCTGCATAACCTT-3' (SEQ ID NO:8).

Preferred primers used when methionine at the 18-position is changed to leucine include

5′-ACATTTGAACTCGCTGGAGGGTGTAGAA-3′ (oligonucleotide
primer B) (SEQ ID NO:9).

Primers for changing the above site to isoleucine include

5'-ACATTTGAACTCGATCGAGGGTGTAGAA-3' (SEQ ID NO:10).

When the site is changed to threonine, primers include

5'-ACATTTGAACTCGACGGAGGGTGTAGAA-3' (SEQ ID NO:11).

Preferred primers used when phenylalanine at the 34-position is changed to cysteine include

5'-GATGTGCACAATTGTGTTGCCTTAGGTGCC-3' (SEQ ID NO:12).

Further, preferred primers used site-directed mutagenesis, respectively include 
5'-CACAATTTTTGCGCCTTAGG-3' (oligonucleotide primer A)
(SEQ ID NO:13),
when valine at the 35-position is changed to cysteine, 5'-AATTTTGTTGCCTGTGGTGCCCCATTG-3' (SEQ ID NO:14), when leucine at the 37-position is changed to cysteine, 5'-GTTGCCTTAGGTTGCCCATTGGCTCCT-3' (SEQ ID NO:15), when alanine at the 39-position is changed to cysteine, 5'-TTAGGTGCCCCATGTGCTCCTCGTCAT-3' (SEQ ID NO:16), when leucine at the 41-position is changed to cysteine, 5'-GCCCCATTGGCTTGTCGTGATGCTGGT-3' (SEQ ID NO:17), when proline at the 43-position is changed to cysteine, and 5'-CCATTGGCTCCTTGTGATGCTGGTTCC-3' (SEQ ID NO:18), when arginine at the 44-position is changed to cysteine.

Other nucleotide primers can be made by those skilled in the art using conventional techniques.

The primer is hybridized to a single-stranded phage in which a single strand of the human PTH gene is cloned, such as M13 [Yanisch-Perror, C. Vieira and J. Messing, Gene, 33, 103-119 (1985); J. Messing, Methods in Enzymology, 101, 20-78 (1983)], fd [R. Herrman et al., Mol. Gen. Genet., 177, 231 (1980)] or φX174 [M. Smith and S. Gillam, Genetic Engineering, Plenum Press, vol.3, p.1-32 (1981)], or to a chimera vector of a phage and a plasmid such as pUC118 or pUC119 [J. Vieira and J. Messing, Methods in Enzymology, 153, 3-11 (1987)]. It is observed that the phage can carry both a sense chain and an anti-sense chain of the gene. When the phage carries the anti-sense chain, in addition to discrepancy from the codon determining a triplet which has encoded another amino acid, the primer may not be the same as a sense chain region containing a codon to which mutation is to be induced, due to codon degeneracy. Similarly, when the phage carries the sense chain, the primer may not be complementary to the sense chain region containing a codon to which mutation is to be induced, as well as appropriate discrepancy from a triplet which pairs to a codon to be deleted. The conditions used for hybridization are described by M. Smith and S. Gillam in the above literature. The temperature is usually within the range from 0 to 70°C, and more generally within the range from 10 to 50°C. After hybridization, the primer is elongated on a phage DNA by reaction with E. coli DNA polymerase I, T4 DNA polymerase, a reverse transcriptase or another suitable DNA polymerase. The resulting double-stranded DNA (dsDNA) is converted to a closed circular dsDNA by treatment with a DNA ligase such as T4 DNA ligase. DNA molecules containing single-stranded regions can be decomposed by S1 endonuclease treatment.

The resulting mutational heteroduplex is used for transformation of infectable host organisms or cells. In the replication of the heteroduplex by using the host, progenies are produced from both chains. Following the replication, a mutant gene is isolated from the progeny of the mutant chain, and inserted into an appropriate vector. The resulting vector is used for transformation of appropriate host organisms or cells.

Then, the phage DNA carrying the mutational gene is isolated, and incorporated into a plasmid.

Examples of the plasmids into which DNAs are incorporated include plasmids derived from E. coli, such as pBR322 [Gene 2, 95 (1977)], pBR325 [Gene 4, 121 (1978)], pUC12 [Gene 19, 259 (1982)] and pUC13 [Gene 19, 259 (1982)]; and plasmids derived from Bacillus subtilis, such as pUB110 [Biochemical and Biophysical Research Communication, 112, 678 (1983)]. However, any other plasmid may be used, as long as it is replicable and maintainable in the host.

Examples of methods for incorporating the phage DNA into the plasmid include the method described in T. Maniatis et al., Molecular Cloning, Cold Spring Harbor Laboratory, p.239 (1982).

The genes coding for peptides lacking N-terminal regions of human PTH (1-84) include, for example, genes having sequences (SEQ ID NOs: 19 to 22) shown in Fig. 1, and these genes can also be obtained by synthesis. In Fig. 1, amino acid sequences are also shown in addition to DNA sequences used in the present invention. In addition, a gene coding for human PTH (1-84) and its amino acid sequence (SEQ ID NO:23) are also shown in Fig. 1.

When the above genes are used for expression, an initiation codon ATG and a stop codon (for example, TAA) are directly arranged on the 5'- and 3'-terminal sides, respectively, of the sequence of each polypeptide lacking the N-terminal region of human PTH, and for example, NdeI and BamHI are ligated to the 5'- and 3'-termini, respectively, for insertion into a vector, as shown in Fig. 2 (SEQ ID NOs: 24 to 31). In Fig. 2 are also shown sequences (SEQ ID NOs: 32 and 33) in which the above-mentioned initiation codon and stop codon are added to the gene coding for human PTH (1-84).

In synthesizing the human PTH-related gene of the present invention, its structural gene is finally cleaved into 14 fragments, for example, as shown in Fig. 2. The respective DNA fragments are shown in Fig. 3, and fragments #1-e, #2-e and #3 to #14 (SEQ ID NOs: 42 to 55) were already provided by the present inventors (European Unexamined Patent Publication No. 477885).

Methods for cleaving the gene into the fragments are not required to be limited to the above-mentioned method, and various methods are also available as long as the method avoids self association.

Fragments #1 to #14 (SEQ ID NOs: 34 to 55) can be produced by known synthesizing methods. For the fragments except for #1 and #14, the 5'-termini are phosphorylated with polynucleotide kinase as required, and all of the fragments are hybridized at once to ligate them to one another with DNA ligase (Fig. 4), or the phosphorylated fragments first divided into two or three groups are hybridized to form a double-stranded DNA fragment with DNA ligase, and the DNA fragments of the respective groups were further ligated to one another with DNA ligase, thereby obtaining a complete double-stranded human PTH gene.

The resulting gene is ligated to a digested product of pUC19 with NdeI and BamHI to obtain a novel plasmid pU-PTH-C19, and E. coli JM109 is transformed. As to the isolated plasmid, the nucleotide sequence is determined by the Sanger method using a portion of the DNA fragment as a primer. More easily, the DNA fragment obtained by digestion with NdeI and BamHI is digested with AurII, NcoI, HgiAI or AluI to give a correct restriction site, thereby confirming the existence of the human PTH gene lacking the N-terminal portion. Further, this gene can be changed to the derivatives in which leucine is substituted for methionine at the 8- and 18-positions and cysteine is substituted for various amino acid residues at the 34- to 47-positions by site-directed mutagenesis.

The gene thus cloned is ligated downstream from a promoter in a vehicle (vector) suitable for expression, whereby an expression vector can be obtained.

The vectors include the above-mentioned plasmids derived from E. coli (such as pBR322, pBR325, pUC12 and pUC13), plasmids derived from B. subtilis (such as pUB110, pTP5 and pC194), plasmids derived from yeast (such as pSH19 and pSH15), bacteriophages such as λ phage, and animal viruses such as retroviruses and vaccinia viruses.

The gene has ATG as an initiation codon at the 5'-terminus thereof and may have TAA, TGA or TAG as a stop codon at the 3'-terminus. In order to express the gene, a promoter is ligated upstream therefrom. As the promoter used in the present invention, any promoter is available as long as it is suitable for expression corresponding to the host used for the gene expression.

When the host cell used for transformation is Escherichia, it is preferable to use a trp promoter, a lac promoter, a recA promoter, a λPL promoter, a lpp promoter, a pharge T7φ10 promoter. When the host cell is Bacillus, it is preferable to use an SPO1 promoter, an SPO2 promoter or a penP promoter. When the host cell is yeast, it is preferable to use a PHO5 promoter, a PGK promoter, a GAP promoter, an ADH promoter. In particular, it is preferable that the host cell is Escherichia and the promoter is a trp promoter, a λPL promoter or a pharge T7φ10 promoter.

When the host cell is an animal cell, an SV40-derived promoter or a retrovirus promoter can be used. The SV40-derived promoter is preferably used among others.

By using the vector containing the recombinant DNA having a nucleotide sequence coding for the mutein thus constructed, a transformant for carrying the vector is prepared.

The host cells include, for example, Escherichia, Bacillus, yeast and animal cells.

Examples of the cells belonging to the genus Escherichia described above include E. coli K12DH1 [Proc. Natl. Acad. Sci. U.S.A., 60, 160 (1968)], JM103 [Nucleic Acids Research, 9, 309 (1981)], JA221 [Journal of Molecular Biology, 120, 517 (1978)], HB101 [Journal of Molecular Biology, 41, 459 (1969)], C600 [Genetics, 39, 440 (1954)], MM294 [Proc. Natl. Acad. Sci. U.S.A., 73, 4174 (1976)] and MM29 4(DE3)/pLysS (Japanese Patent Unexamined Publication No. 3-43088/1991).

Examples of the cells belonging to the genus Bacillus described above include Bacillus subtilis MI 114 [Gene, 24, 255 (1983)] and 207-21 [Journal of Biochemistry, 95, 87 (1984)].

Examples of the yeast include Saccharomyces cerevisiae AH22R, NA87-11A and DKD-5D.

Examples of the animal cells include monkey cell COS-7, Vero, Chinese hamster cell (CHO), mouse L cell and human FL cell.

The transformation of Escherichia strains described above is conducted, for example, according to the method described in Proc. Natl. Acad. Sci. U.S.A., 69, 2110 (1972), Gene, 17, 107 (1982).

The transformation of Bacillus strains is carried out, for example, according to the method described in Molecular & General Genetics, 168, 111 (1979).

The transformation of yeast is performed, for example, according to the method described in Proc. Natl. Acad. Sci. U.S.A., 75, 1929 (1978).

The transformation of animal cells is carried out, for example, according to the method described in Virology, 52, 456 (1973).

Thus, the transformant transformed with the vector containing the recombinant DNA having the nucleotide sequence coding for the mutein is obtained.

The mutein is produced by cultivating the transformant in a culture medium.

When bacterial transformants are cultivated, a liquid medium is suitably used for cultivation. Carbon sources, nitrogen sources, inorganic compounds and other nutrients necessary for growth of the transformants are contained therein. Examples of the carbon sources include glucose, dextrin, soluble starch and sucrose. Examples of the nitrogen sources include inorganic or organic materials such as ammonium salts, nitrates, corn steep liquor, peptone, casein, meat extracts, soybean meal and potato extract solution. The inorganic compounds include, for example, calcium chloride, dibasic sodium phosphate and magnesium chloride. Yeast, vitamins and growth promoting factors may be further added thereto.

The pH of the medium is preferably about 6 to about 8.

When the Escherichia transformants are cultivated, M9 medium containing glucose and Casamino Acids [Miller, Journal of Experiments in Molecular Genetics, 431-433, Cold Spring Harbor Laboratory, New York (1972)] is preferably used to cultivate the transformants. In order to allow the promoters to act more efficiently, for example, drugs such as 3β-indolyl acrylic acid and isopropyl βD-thiogalactopyranoside may be added thereto if necessary.

The Escherichia transformants are usually cultivated at 15 to 43°C for 3 to 24 hours with aeration or agitation if necessary.

The Bacillus transformants are usually cultivated at 30 to 40°C for 6 to 24 hours with aeration or agitation if necessary.

When the yeast transformants are cultivated, the preferred medium is Burkholder minimum medium [K. L. Bostian, Proc. Natl. Acad. Sci. U.S.A., 77, 4505 (1980)]. The pH of the medium is preferably adjusted to 5 to 8. The cultivation is usually carried out at 20 to 35°C for 24 to 72 hours with aeration or agitation if necessary.

When the animal cell transformants are cultivated, examples of media which can be used include MEM medium containing 0 to 20% fetal calf serum [Science, 122, 501 (1952)], DME medium [Virology, 8, 396 (1959)], RPMI1640 medium [Journal of the American Medical Association, 199, 519 (1967)] and 199 medium [Proceeding of the Society for the Biological Medicine, 73, 1 (1950)]. The pH is preferably 6 to 8. The cultivation is usually carried out at 30 to 40°C for 15 to 60 hours, with aeration or agitation if necessary.

The isolation and purification of the mutein from the above-mentioned culture products can be carried out, for example, according to the following method.

First, the cultivated cells are disrupted to extract the contents. The disruption is conducted by various methods using a French press, ultrasonic waves, lysozyme, freeze-thawing or glass beads. However, any method may be used. When the cells are disrupted, 1-8 M urea or 1-6 M guanidine hydrochloride (Gu-HCl) may be added to a buffer solution. A reducing agent such as dithiothreitol is added to increase the recovery of the desired mutein in some cases. The reducing agent is added after lysozyme has been reacted.

Then, the above-mentioned extract is subjected to centrifugation to separate a supernatant from a precipitate. When the mutein is recovered in the supernatant, it can be effectively purified, for example, according to a method similar to the method described in M. Iwane et al., Biochem. Biophys. Res. Commun., 146, 470-477 (1987). When the mutein is recovered in the precipitate, this precipitate is dissolved in a solution containing a protein denaturant such as urea or guanidine hydrochloride, followed by dialysis or dilution to decrease the concentration of the denaturant, whereby the biologically active mutein can be obtained. The mutein recovered from the precipitate is purified as so desired, which leads to a high purity, high active product similarly with the mutein recovered from the supernatant.

In particular, for the mutein containing Cys, the coexistence of a slight amount of the reducing agent in the purification course or the storage course is suitable for preventing the product from being oxidized. Human PTH can be separated from extracted solutions and purified by the use of known techniques. The separating and purifying techniques include column chromatography such as gel filtration, ion exchange chromatography using a cation exchange resin or an anion exchange resin, column chromatography such as hydrophobic chromatography and partition adsorption chromatography, and high performance liquid chromatography.

The cultivation of the transformants of Bacillus, yeast and animal cells and the separation and purification of human PTH from the culture products are carried out by methods known per se in the art.

The resulting human PTH mutein of the present invention are useful as therapeutic drug. When the resulting human PTH mutein is an agonist derivative, it can be used as therapeutic agents for various diseases caused by the abnormality of calcium metabolism, for example, osteoporosis and hypoparathyroidism, and as therapeutic agents for hypertension. Further, the human PTH antagonist derivatives can be used as therapeutic agents for hypercalcemia and hyperparathyroidism. The dosage thereof is properly determined in each case, taking into account the object of administration, the disease and the like, and a suitable amount is given within the range of 1 ng to 100 µg/kg of weight a day. Usually, human PTH derivative of the present invention is mainly given parenterally in combination with pharmaceutically acceptable carriers, excipients or diluents as injections, nasotracheal agents, perrectum agents, transvaginal agents or percutaneous agents, but it may be given orally in some cases.

The human PTH mutein in which one amino acid residue within the region of amino acid residue Nos. 34 to 47 is substituted by a cysteine residue is subjected to cleavage reaction to cleave peptide bonds on the amino group side of the cysteine residue and can be used for the production of various fragments different in chain length which have the biological activity of human PTH.

Examples of the cleavage reaction include cyanylation, hydrolysis or aminolysis.

The cyanylation reaction is conducted by reacting an S-cyanylating reagent with a partially purified material compound.

Examples of the S-cyanylating reagents include 2-nitro-5-thiocyanobenzoic acid (NTCB), 1-cyano-4-dimethylamino-pyridinium salts (DMPA-CN) and CN⁻ ions. The amount of the S-cyanylating reagents used is twice to 50 times as much as all thiol groups, and more preferably, 5 to 10 times.

The reaction temperature may be any as long as it is within the range of 0 to 80°C. A temperature of 0 to 50°C is more preferably used. Any buffer may be used as a solvent as long as it does not react with the cyanylating reagent. Examples of such buffer solutions include Tris-HCl buffer, Tris-acetate buffer, phosphate buffer and borate buffer. An organic solvent may be used as long as it does not react with the cyanating reagent.

The reaction is carried out in pH range 1 to 12. Further, pH 7 to 10 is preferable when NTCB is used. And pH 2 to 7 is preferable to prevent S-S exchange reaction when DMAP-CN is used. Denaturation agent, such as guanidinechloride, may be used in the reaction solution.

The above-mentioned hydrolysis or aminolysis is conducted by subjecting the starting material product to alkali treatment. The alkali treatment is carried out by adjusting a solution containing the starting material to pH 7-14.

The adjustment of the pH is performed, for example, by adding an appropriate amount of a solution of sodium hydroxide, ammonium, a substituted amino compound, trituma base [Tris(hydroxymethyl)-aminomethane], sodium dihydrogenphosphate, potassium hydroxide or barium hydroxide to the solution containing the cyanated compound. The substituted amino compounds include the above-mentioned compounds.

The concentration of the solution in the above-mentioned reaction is, for example, 0.01 to 2 N, preferably 0.1 to 1 N for sodium hydroxide, 0.01 to 15 N, preferably 0.1 to 3 N for ammonium or substituted amino compounds, 1 mM to 1 M, preferably 20 mM to 200 mM for trituma base, 1 mM to 1 M, preferably 10 mM to 100 mM for sodiumic phosphate, 0.01 to 4 N, preferably 0.1 to 2 N for potassium hydroxide, and 0.01 to 0.2 M, preferably 0.1 to 0.2 M for barium hydroxide. The reaction temperature may be any as long as it ranges from 0°C to 80°C. A temperature of 0 to 50°C is more preferably used.

The reaction time is preferably 1 to 60 minutes, more preferably 15 to 30 minutes for cyanylation reaction, 5 minutes to 100 hours, preferably 10 minutes to 15 hours for hydrolysis, and 5 minutes to 24 hours, preferably 10 to 180 minutes for aminolysis.

The reaction shown in Fig. 33 is considered to take place by the above-mentioned cyanylation and hydrolysis or aminolysis. In Fig. 33, X represents OH or R-NH-(wherein R-NH- represents an amino group or a substituted amido group). In this reaction, when ammonium or the substituted amino compound is used, a corresponding amide compound or substituted amide compound is obtained.

The peptide fragments cut out are isolated according to known methods for purifying peptides, for example, by suitable combinations of gel filtration, ion-exchange chromatography, high performance liquid chromatography, affinity chromatography hydrophobic chromatography, thin layer chromatography and electrophoresis. Methionine derived from the initiation codon is sometimes attached to the N-terminus of the peptide fragment obtained here.

The resulting peptide fragments may be lyophilized if necessary. In lyophilization, stabilizers such as sorbitol, mannitol, dextrose, maltose, trehalose and glycerol may be added.

In the present invention, for human PTH (1-84) and the antagonists lacking its N-terminal amino acid sequences, the respective genes are subjected to site-directed mutagenesis, and the methionine residues are changed to other lipophilic residues by expression of the resulting modified genes, thereby forming the anti-oxidative muteins. Further, the muteins in which various amino acid residues in the central portions of the polypeptide are changed to cysteine are prepared. Such muteins can form dimers. Furthermore, various substituent groups (for example, lipophilic alkyl groups) are introduced into the side chains of the cysteine residues, whereby the human PTH agonist muteins and antagonist muteins can be stabilized, the activity can be enhanced, and the absorption to the tissues can be improved. Thus, the present invention provides the human PTH muteins useful for clinical application.

When nucleotides, amino acids and so on are indicated by abbreviations in the specification and drawings, the abbreviations adopted by the IUPAC-IUB Commission on Biochemical Nomenclature or commonly used in the art are employed. For example, the following abbreviations are used. When the amino acids are capable of existing as optical isomers, it is understood that the L-forms are represented unless otherwise specified.
- DNA :: Deoxyribonucleic acid
- A :: Adenine
- T :: Thymine
- G :: Guanine
- C :: Cytosine
- RNA :: Ribonucleic acid
- dATP :: Deoxyadenosine triphosphate
- dTTP :: Deoxythymidine triphosphate
- dGTP :: Deoxyguanosine triphosphate
- dCTP :: Deoxycytidine triphosphate
- ATP :: Adenosine triphosphate
- Tdr :: Thymidine
- EDTA :: Ethylenediaminetetraacetic acid
- SDS :: Sodium dodecyl sulfate
- Gly or G :: Glycine
- Ala or A :: Alanine
- Val or V :: Valine
- Leu or L :: Leucine
- Ile or I :: Isoleucine
- Ser or S :: Serine
- Thr or T :: Threonine
- Cys or C :: Cysteine
- Met or M :: Methionine
- Glu or E :: Glutamic acid
- Asp or D :: Aspartic acid
- Lys or K :: Lysine
- Arg or R :: Arginine
- His or H :: Histidine
- Phe or F :: Phenylalanine
- Tyr or Y :: Tyrosine
- Trp or W :: Tryptophan
- Pro or P :: Proline
- Asn or N :: Asparagine
- Gln or Q: Glutamine

The present invention will be described in more detail through following reference examples and examples. It is understood of course that these examples are not intended to limit the scope of the invention.

Transformants E. coli MM294(DE3)/pE-L8PTH and E. coli MM294(DE3)/pE-C35PTH obtained in examples described below were deposited with the Institute for Fermentation, Osaka, Japan (IFO) under the accession numbers IFO 15214 and IFO 15213, respectively, on August 7, 1991, and with the Fermentation Research Institute (FRI), the Agency of Industrial Science and Technology, the Ministry of International Trade and Industry, under the accession numbers FERM BP-3507 and FERM BP-3508 on August 22, 1991, under the Budapest Treaty. Similarly, E. coli MM294(DE3)/pE-L8PTH(7-84) was deposited with the IFO under the accession number IFO 15333 on June 3, 1992, and with the FRI under the accession number FERM BP-3886 on June 8, 1992, under the Budapest Treaty.

### Reference Example 1

### Synthesis of DNA Oligomers

Structural gene DNA fragments (#1-c, #2-c and #3 to #14, Fig. 3) (sequence Nos. 38, 39 and 44 to 55) and primers A and B for site-directed mutagenesis (sequence Nos. 9 and 13) were synthesized using properly protected DNA β-cyanoethylphosphoamidite as a starting material with an automatic synthesizer (Model 380A, Applied Biosystems). As a protocol for synthesis, one specified by Applied Biosystems was used. The protected DNA oligomer-resins thus synthesized were heated in 2 ml of concentrated aqueous ammonia based on 0.2 µmole of the resin at 60°C for 6 hours. The resulting products were purified by reversed phase high performance liquid chromatography (hereinafter briefly referred to as HPLC) to obtain DNA oligomers only the 5'-terminal hydroxyl groups of which were protected by dimethoxytrityl groups. These DNA oligomers were treated with 2 ml of 80% acetic acid for 20 minutes to remove the terminal dimethoxytrityl groups, and the resulting products were purified by reverse phase HPLC and ion exchange HPLC.

### Reference Example 2

### Phosphorylation of DNA Oligomers

Of the DNA oligomers obtained in Reference Example 1, each of the twelve DNA oligomers #2-c and #3 to #13 (sequence Nos. 39 and 44 to 54) except for #1-c (sequence No. 38) and #14 (sequence No. 55) which were to form the 5'-termini was reacted in 25 µl of a phosphorylation reaction solution [10 µl of the DNA oligomer, 50 mM Tris-HCl (pH 7.6), 10 mM MgCl₂, 1 mM spermidine, 10 mM dithiothreitol (hereinafter briefly referred to as DTT), 0.1 mg/ml bovine serum albumin (hereinafter briefly referred to as BSA, 1 mM ATP, 10 units of T4 polynucleotide kinase (Takara Shuzo)] at 37°C for 1 hour to phosphorylate the 5'-terminus. This reaction solution was treated at 65°C for 10 minutes, followed by freezing and thawing. The resulting product was subjected to the subsequent reaction. The primers used for site-directed mutagenesis were similarly phosphorylated.

### Reference Example 3

### Construction of Plasmid pU-PTH Containing Human PTH Gene for Site-Directed Mutagenesis

Plasmid pE-PTH (refer to European Unexamined Patent Publication No. 483509) into which the DNA of human PTH was incorporated was digested with BamHI and XbaI to obtain a 0.3-kb DNA fragment containing the DNA of human PTH and an expression vector of pET3c. Then, plasmid vector pUC118 for preparing a single strand was digested with BamHI and XbaI, and mixed with the above-mentioned DNA fragment containing the human PTH gene to ligate them to each other with T4 DNA ligase. Using the DNA thus ligated, E. coli MV1184 was transformed, and seeded onto a plate using Xgal as an indicator species. Recombinant plasmid pU-PTH in which the human PTH gene was correctly inserted into pUC118 was released to a culture medium in the form of phage particles. This single-stranded DNA was purified and used as a template for site-directed mutagenesis. E. coli MV1184 and helper phage KO7 are described in J. Vieira and J. Messing, Methods in Enzymology, 153, 3-11 (1987).

### Reference Example 4

### Production of Human PTH (1-34) OH from [Cys³⁵] Human PTH (1-84)

In 2.4 ml of 6 M guanidine hydrochloride (hereinafter also briefly referred to as Gu-HCl)-0.2 M Tris-acetate buffer (pH 8.0) was dissolved 4.76 mg of [Cys³⁵] human PTH (1-84) obtained in Example 1, and 0.154 mg of dithiothreitol dissolved in 0.1 ml of the above-mentioned buffer was added thereto, followed by standing at room temperature for 30 minutes. Thereto was added 1.646 mg of NTCB dissolved in 0.1 ml of the same buffer, and immediately, the pH was adjusted to 8.0, followed by reaction at room temperature for 15 minutes. After termination of the reaction, 2.5 ml of acetic acid was added, and desalting was conducted by gel filtration using a Sephadex G-25 column. The conditions of gel filtration were as follows:
Column size: 2.6X37 cm
Detecting wavelength: 280 nm
Solvent: 10% acetic acid
Flow rate: 20 ml/hour
Fractions containing [SCN-Cys³⁵] human PTH (1-84) were collected, and subjected to the cleavage reaction after lyophilization.

The cleavage reaction for obtaining human PTH (1-34) OH was conducted in the following manner. Namely, 200 µg of [SCN-Cys³⁵] human PTH (1-84) was allowed to be reacted in 200 µl of 6 M Gu-HCl-0.1 M borate buffer at 37°C for 17 hours, and the same amount of glacial acetic acid was added thereto to terminate the reaction. The reaction solution was analyzed by reverse-phase HPLC (Fig. 30) under the following conditions:
Column: YMCA-303 ODS 4.6X250 mm
Column temperature: 25°C
Elution solvent A: 0.1% TFA-99.9% distilled water
Elution solvent B: 0.1% TFA-99.9% acetonitrile
Elution program: 0 minute (75% A + 25% B), 40 minutes (60% A + 40% B), 45 minutes (20% A + 80% B)
Flow rate: 0.7 ml/minute
Detecting wavelength: 230 nm
In Fig. 30, the peak at a retention time of about 35 minutes indicated by the arrow agreed with the elution time of standard human PTH (1-34) OH purchased from Peptide Laboratory (Japan). This peak fraction was taken and subjected to various protein chemical analyses. Of the cleaved fragments, fragments on the C-terminal side were eluted in fractions not adsorbed to the column, under the elution conditions of this reverse-phase HPLC.

The sample was subjected to the hydrochloric acid hydrolysis process (hydrolyzed with 5.7 N hydrochloric acid in the presence of thioglycollic acid in a sealed tube under reduced pressure at 110°C for 24 hours), and analyzed with a 6380 type amino acid analyzer (Beckman). The amino acid composition values of human PTH (1-34) OH are as shown in Table 1, and these values satisfactorily agree with the theoretical values thereof. Furthermore, it was confirmed by the following method that the carboxyl terminal amino acid Phe³⁴ of human PTH (1-34) OH was not racemized.

The hydrolyzed product used for amino acid analysis was used as a sample, and all amino acids contained in the hydrolyzed product were prelabeled with o-phthalaldehyde. The prelabeled sample was analyzed by reverse-phase HPLC using YMCA-303 ODS (4.6X250 mm) as a column, and an eluent was 50 mM sodium acetate-40% methanol. As a result, the Phe residues contained in the hydrolyzed product were all detected as L-Phe, and no peak of D-Phe was detected at all. Further, the molecular weight of the human PTH (1-34) OH was measured by fast atom bombardment mass spectrometry (FAB-MS). As a result, mass (m/z):(M+H)+ = 4116.8 was observed. This value was satisfactorily agreed with the theoretical value 4118.1 was within the range of an error.

**Table 1**

| Amino Acid Composition of Human PTH(1-34)OH | | |
|---|---|---|
| | Experimental Value | Theoretical Value |
| Asp & Asn | 4.00 | 4 |
| Ser | 2.59 | 3 |
| Glu & Gln | 5.01 | 5 |
| Gly | 1.14 | 1 |
| Val | 2.84 | 3 |
| Met | 1.94 | 2 |
| Ile | 0.85 | 1 |
| Leu | 4.84 | 5 |
| Phe | 0.87 | 1 |
| Lys | 2.94 | 3 |
| His | 2.58 | 3 |
| Trp | 0.65 | 1 |
| Arg | 1.79 | 2 |

### Reference Example 5

### Production of Human PTH (1-34) NH₂ from [Cys³⁵] Human PTH (1-84)

The S-cyanylation of Cys³⁵ of [Cys³⁵] human PTH (1-84) was carried out in the following manner in accordance with the method described in J. C. S. Chem. Comm., 1967, 21-22. In 3.78 ml of 7 M urea-0.1 M ammonium acetate (pH 3.5) was dissolved 8.40 mg of [Cys³⁵] human PTH (1-84), and the solution was allowed to stand at 25°C for 15 minutes. Then, 592 µg of 1-cyano-4-dimethylaminopyridinium fluoroborate dissolved in 0.42 ml of the above-mentioned buffer was added thereto, followed by reaction at room temperature for 15 minutes. Immediately after termination of the reaction, desalting was conducted using a Sephadex G-25 column. The column conditions were as follows:
Column size: 2.6X37 cm
Elution solvent: 10% acetic acid
Flow rate: 20 ml/hour
Detecting wavelength: 280 nm
Fractions containing [SCN-Cys³⁵] human PTH (1-84) were collected and lyophilized. The yield was 7.5 mg. The resulting product was used for the following cleavage reaction.

In 200 µl of 3 M aqueous ammonia was dissolved 200 µg of [SCN-Cys³⁵] human PTH (1-84), followed by reaction at 37°C for 10 minutes. The reaction solution was analyzed by reverse-phase HPLC under the conditions shown in Reference Example 4. As shown in Fig. 31, [SCN-Cys³⁵] human PTH (1-84) completely disappeared, and one peak having a shoulder at a retention time of 32 minute was observed. A main peak portion of this peak agreed with the elution position of standard human PTH (1-34) NH₂ obtained by solid phase synthesis. Of the cleaved fragments, fragments on the C-terminal side were eluted in flow-through fractions.

### Reference Example 6

### Production of Human PTH (1-34) NHC₂H₅ from [Cys³⁵] Human PTH (1-84)

In 500 µl of 3.1 M ethylamine was dissolved 200 µg of [SCN-Cys³⁵] human PTH (1-84) obtained in Reference Example 4, followed by reaction at 37°C for 20 minutes. Then, the same amount of glacial acetic acid was added thereto to terminate the reaction. The resulting reaction solution was analyzed by reverse-phase HPLC under the conditions shown in Reference Example 3. As shown in Fig. 32, [SCN-Cys³⁵] human PTH (1-84) disappeared, and two peaks were mainly detected. Peaks eluted at retention times of 31 minutes and 36 minutes were named "peak 7" and "peak 8", respectively. Each fraction was taken and subjected to the protein chemical analyses.

The amino acid composition values of the peak 8 fraction which were analyzed according to the method described in Reference Example 3 are as shown in Table 2, and these values satisfactorily agree with the theoretical values of human PTH (1-34) NHC₂H₅. Furthermore, using as a sample the hydrolyzed product used for amino acid analysis, it was confirmed whether the carboxyl terminal amino acid Phe³⁴ of the peak 8 fraction was the D-form or the L-form.

All amino acids contained in the sample were prelabeled with o-phthalaldehyde, and then, the prelabeled sample was analyzed by reverse-phase HPLC using a YMCA-303 ODS column (4.6X250 mm). An eluent was 50 mM sodium acetate-40% methanol. As a result, the Phe residues contained in the hydrolyzed product were all detected as L-Phe, and no peak of D-Phe was detected at all. Further, the molecular weight of the resulting human PTH (1-34) NHC₂H₅ was measured by FAB-MS. As a result, mass (m/z):(M+H)+ = 4144.9 was observed. This value was satisfactorily agreed with the theoretical value 4143.2 was within the range of an error.

As to the peak 7 fraction, amino acid analysis was similarly conducted. The composition ratio obtained practically agreed with that of [SCN-Cys³⁵] human PTH (1-84). The raw material, [SCN-Cys³⁵] human PTH (1-84), is eluted at a retention time of about 35 minutes. This shows that peak 7 is [dehydroalanine³⁵] human PTH (1-84) produced by β elimination of the S-cyano group of [SCN-Cys³⁵] human PTH (1-84). Y. Degani, A. Patchornik, et al. report that the β elimination reaction takes place, competing with the cleavage reaction [Biochemistry, 13, 1-11 (1974)].

Of the cleaved fragments, fragments on the C-terminal side were eluted in flow-through fractions under the above-mentioned conditions.

**Table 2**

| Amino Acid Composition of Human PTH (1-34) NHC₂H₅ | | |
|---|---|---|
| | Experimental Value | Theoretical Value |
| Asp & Asn | 4.01 | 4 |
| Ser | 2.74 | 3 |
| Glu & Gln | 5.22 | 5 |
| Gly | 1.35 | 1 |
| Val | 2.74 | 3 |
| Met | 2.09 | 2 |
| Ile | 0.91 | 1 |
| Leu | 4.86 | 5 |
| Phe | 1.00 | 1 |
| Lys | 2.89 | 3 |
| His | 2.58 | 3 |
| Trp | 0.75 | 1 |
| Arg | 1.43 | 2 |
| Ethylamine | 1.58 | 1 |

### Reference Example 7

### Production of Gene for Coding Human PTH (5-84) and Expression Thereof

### (1) Ligation of DNA Fragments (refer to Fig. 4)

Series of stages for forming a double-stranded structure of a human PTH gene are as shown in Fig. 4. Referring to Fig. 4, the mark 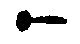 indicates that a 5'-terminal hydroxyl group is phosphorylated. Each of the phosphorylated reaction solutions of the twelve DNA fragments [corresponding to DNA fragments #2-c and #3 to #13 (SEQ ID NOs: 39 and 44 to 54)] shown in Fig. 3 were combined in an amount of 5 µl with 2 µg of DNA fragment #1-c (SEQ ID NO: 38) and #14 (SEQ ID NO: 55) corresponding to the 5'-terminus to 70 µl. Then, 5 units of T4 DNA ligase (Takara Shuzo) was added thereto, followed by incubation at 15°C for 20 hours.

The product thus obtained was subjected to electrophoresis on a 8% polyacrylamide gel in a buffer (pH 8.3, 100 mM Tris-HCl, 100 mM borate, 2 mM EDTA) at 125 V for 2 hours. After electrophoresis, the gel was stained with 0.6 mg/l EtBr. Gel bands containing 263-bp DNA fragments were sealed in a dialysis tube and submerged in a buffer for electrophoresis. Then, the DNA fragments were electrically eluted from the gel. A solution in this dialysis tube was treated with phenol twice, followed by recovery of an aqueous layer (an upper layer). Then, twice as much ethanol as the aqueous layer was added thereto, and the mixture was cooled to -70°C. The DNA fragments were thereafter precipitated by centrifugation. Thus, about 1 µg of the DNA fragments was obtained. After phosphorylation with T4 polynucleotide kinase (Takara Shuzo), the DNA fragments were subjected to the following experiment (2).

### (2) Cloning of Human PTH (5-84) Gene (Fig. 5)

As a cloning vector, E. coli plasmid pBR322-derived pUC19 [J. Messing, Gene, 33, 103-109 (1985)] was used. pUC19 DNA was digested in 20 µl of a reaction solution [20 mM Tris-HCl (pH 7.6), 7 mM MgCl₂, 150 mM NaCl, 10 mM 2-mercaptoethanol, 20 units of NdeI (New England Biolabo), 15 units of BamHI (Takara Shuzo)] at 37°C for 24 hours. Then, the resulting product was diluted 5 times with water, and treated at 65°C for 20 minutes to inactivate the enzyme. 5 µl of this reaction solution was mixed with about 5 equivalents of the DNA fragments obtained in (1) described above to prepare 20 µl of a reaction solution containing 50 mM Tris-HCl (pH 7.5), 10 mM MgCl₂, 10 mM DTT, 1 mM spermidine, 0.1 mg/ml BSA and 1 mM ATP. Then, T4 DNA ligase (New England Biolabo) was allowed to be reacted with this solution at 14°C for 15 hours to ligate the human PTH (5-84) gene to the plasmid.

Using this reaction solution, the E. coli JM109 strain [J. Messing, Gene, 33, 103-119 (1985)] was transformed according to methods known in the art. Namely, 50 µl of competent cells [D. Hanahan, J. Mol. Biol., 166, 557 (1983)] stored at -70°C was incubated at 0°C for 15 minutes, and then 10 µl of the above-mentioned reaction solution was added thereto. The resulting solution was further incubated at 0°C for 30 minutes, and then incubated at 42°C for 1.5 minutes and further at 0°C for 2 minutes. To this reaction solution was added 200 µl of LB medium (containing 10 g of bactotryptone, 5 g of a bactoyeast extract and 5 g of NaCl per 1 liter), followed by incubation at 37°C for 1 hour. This E. coli was seeded onto LB agar medium (Lurla-Bertant Medium) (bacto-triptone 10g/l, bacto-yeast extract 5g/l, NaCl 10g/l) containing 50 µg/ml ampicillin, 100 µg/ml X-Gal and 0.1 mM isopropyl-β-D-thiogalactopyranoside (hereinafter referred to as IPTG), and incubated at 37°C overnight. Of the resulting ampicillin-resistant colonies, 14 β-galactosidase-deficient strains were selected and plasmid DNAs of transformed strains thereof were crudely purified by the alkali method [T. Maniatis et al., Molecular Cloning, (Cold Spring Harbor Laboratory) 368-369 (1982)], followed by digestion with NcoI and BamHI and further with NdeI and BamHI. The electrophoresis patterns of these digests on a 1.7% agarose gel revealed that one strain was a transformed strain into which the human PTH (5-84) gene (SEQ ID:Nos: 28 and 29) was correctly inserted.

### (3) Construction of Plasmid for Expression of Human PTH (5-84) and Production of Transformant (Fig. 5)

(i) About 10 µg of pU-PTH(5-84)-19 obtained in the above experiment (2) was digested in a reaction solution [150 mM NaCl, 20 mM Tris-HCl (pH 7.8), 7 mM MgCl₂, 10 mM mercaptoethanol, 40 units of NdeI, 20 units of BamHI (Takara Shuzo)] at 37°C for 5 hours. Then, 263-bp DNA fragments were purified by 1.7% agarose gel electrophoresis according to known methods. On the other hand, as a vector for expression, pET3c [F. W. Stadier et al., Methods in Enzymology 195, 60-89 (1990)] was used. pET3c DNA was digested with NdeI and BamHI in the same manner as above, and four times as much water as the resulting reaction solution was added thereto, followed by heating at 65°C for 20 minutes to inactivate the enzyme.

Each of the 263-bp DNA and the plasmid DNA has single-stranded attachment termini produced by NdeI digestion and BamHI digestion at both ends thereof.

Both of them were mixed with each other, and the mixture was reacted with T4 DNA ligase (New England Biolabs) in the presence of 50 mM Tris-HCl (pH 7.6), 10 mM MgCl₂, 10 mM DTT, 1 mM spermidine, 0.1 mg/ml BSA and 1 mM ATP at 14°C for 16 hours to ligate the DNAs to each other, followed by transformation of the E. coli JM109 strain in the same manner as above. Then, this E. coli was seeded onto LB agar medium containing 50 µg/ml ampicillin, and cultivated at 37°C for 1 day. The resulting ampicillin-resistant colony was selected. A plasmid DNA of the transformed strain was further digested by a combinations of restriction enzymes such as NdeI-BamHI, BglII-BamHI, EcoRI-NdeI or AvrII-BglII. The transformed strain correctly containing the human PTH gene was selected by its pattern of polyacrylamide electrophoresis. The plasmid for expression thus obtained was named pE-PTH (5-84), and the transformed strain was named JM109/pE-PTH (5-84).

(ii) The plasmid DNA was isolated from JM109/pE-PTH (5-84) obtained in (i), and crudely purified. Then, E. coli MM294(DE3) prepared by lysogenizing λ phage DE3 [F. W. Studier et al., J. Mol. Biol., 189, 113 (1986)] in which an RNA polymerase gene of T7 phage was incorporated into E. coli MM294 was transformed. First, 10 ml of LD medium was inoculated with one loopful of E. coli MM294(DE3), and cultivated at 37°C with shaking to a Klett of 60 to 180. To 50 µl of this culture solution were added 10% w/v polyethylene glycol, 5% v/v dimethyl sulfoxide and 50 mM MgCl₂ (pH 6.5), and a reaction solution was brought up to 100 µl with the addition of LB medium. The plasmid DNA was added thereto in an amount of 10 ng, and incubated at 4°C for 10 minutes, followed by seeding onto LB agar medium containing 50 µg/ml ampicillin. Then, cultivation was carried out overnight at 37°C.

The plasmid DNA obtained from the resulting colony similarly with the method described above was digested with restriction enzymes, and the transformed strain containing the human PTH gene was selected by its pattern of electrophoresis. This strain was named E. coli MM294(DE3)/pE-PTH (5-84).

### (4) Expression of Human PTH (5-84)

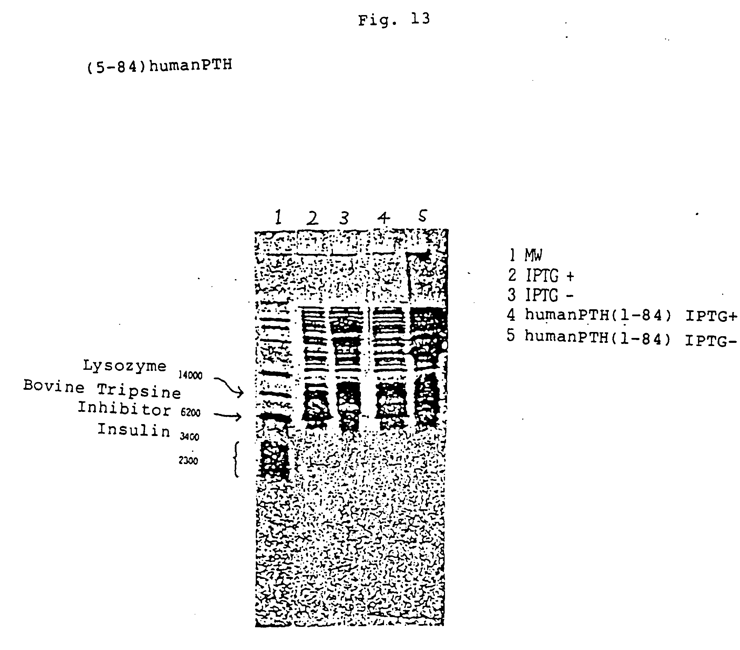

E. coli MM294(DE3)/pE-PTH (5-84) was cultivated overnight at 37°C in LB medium containing 50 µg/ml ampicillin with shaking. To 10 ml of the same medium dispensed into a 200-ml flask, 100 µl of the resulting culture solution was added and cultivated at 37°C to a Klett of about 170. Then, IPTG was added thereto to a concentration of 0.1 mM. After cultivation was further continued for 2 hours, 1 ml of the culture solution was centrifuged at 15,000 rpm at 4°C for 5 minutes. The resulting cells were dissolved in 100 ml of a solution containing 0.5 M Tris-HCl (pH 6.8), 10% glycerol, 10% (w/v) sodium dodecyl sulfate (SDS), 0.1% (w/v) β-mercaptoethanol and bromophenol blue [U. K. Laemmli, Nature, 227, 680 (1970). After boiling for 3 minutes, the solution was subjected to 16% SDS-polyacrylamide gel electrophoresis (PAGE). After electrophoresis, the gel was stained with Coomassie Brilliant Blue. As a result, an intense band indicating a mobility approximately similar to that of a standard human PTH sample was observed (refer to Fig. 13). In the figure 13, Lanes 1 to 5 are as follows:
Lane 1: Molecular weight marker
Lane 2: E. coli strain culture solution (10 µl) carrying plasmid pE-PTH (5-84) after induction with IPTG
Lane 3: E. coli strain culture solution (10 µl) carrying plasmid pE-PTH (5-84) without induction with IPTG
Lane 4: Human PTH expression strain culture solution (10 µl) after induction with IPTG
Lane 5: Human PTH expression strain culture solution without induction with IPTG.

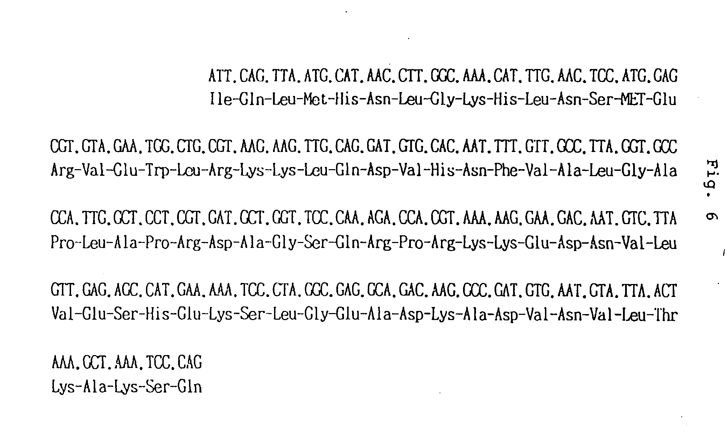

From the quantitative comparison with the standard sample in gel staining, human PTH (5-84) was expressed in an amount of about 100 mg/l. Thus, human PTH (5-84) having the amino acid sequence shown in Fig. 6 (sequence No. 21) was obtained.

### Example 1

### Production of Gene Coding for [Cys³⁵] Human PTH (1-84) and Expression Thereof

### (1) Production of Gene Coding for [Cys³⁵] Human PTH (1-84) (Fig. 7)

First, oligonucleotide primer A, CACAATTTTTGCGCCTTAG-GTGC (SEQ ID:NO: 13), was synthesized to change a codon of Val at the 35-position to a codon of Cys. Using the above-mentioned synthetic oligonucleotide (4 picomols) in which the 5' OH terminus was phosphorylated by treatment with T4 kinase and the single-stranded pU-PTH (5 µg) previously mentioned, a plasmid was obtained into which a mutation was introduced with a site-directed mutagenesis kit (oligonucleotide-directed in vitro mutagenesis system version 2, Amersham). E. coli MV1184 was normally transformed by this plasmid and seeded on a double YT medium (bacto-triptone 16g/l, bacto-yeast extract 10g/l, NaCl 5g/l) agar plate containing 150 µg/ml ampicillin. Then, cultivation was carried out at 37°C for 15 hours to obtain many colonies. Of these, a small amount of cells were collected from 10 colonies, and cultivated on 0.3 ml of double YT medium for about 5 hours. Thirty µl of this culture solution was mixed with 30 µl of a solution containing helper phage KO7, and the mixture was allowed to stand at 37°C for 1 hour. Then, 3 ml of double YT medium was added thereto, followed by cultivation overnight. The resulting culture solution was subjected to centrifugation to separate a supernatant from cells. A plasmid was crudely purified from the cell by the alkali method, and a single-stranded DNA existing as a pharge particle was recovered from the supernatant.

The above-mentioned oligonucleotide primer A contains recognition sites for restriction enzyme HhaI which do not exist in the gene coding for human PTH, said gene functioning as a template.

Accordingly, when HhaI is reacted with the plasmid into which a mutation is correctly introduced, the plasmid must be cleaved at 25 sites of the HhaI sites newly generated by the mutation and HhaI sites originally existing in pUC118 to produce 260-bp fragments. The plasmid obtained from the above-mentioned 10 colonies was digested with HhaI, and analyzed by agarose gel electrophoresis. As a result, fragments of correct size were observed in 4 clones.

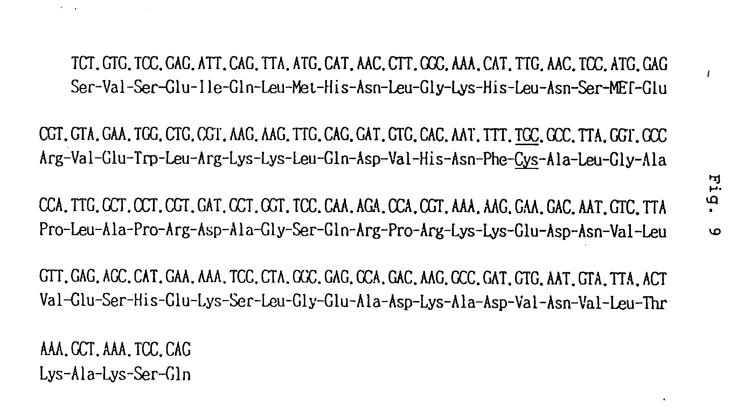

Further, using the single-stranded plasmids of these two clones as templates, the nucleotide sequence was analyzed with a DNA sequencer Model 373A (Applied Biosystems). As a result, the introduction of the desired mutation was confirmed (Fig. 9) (SEQ ID NO: 56).

The thus-obtained plasmid containing the gene (Fig. 9) coding for [Cys³⁵] human PTH was named pU-C35PTH.

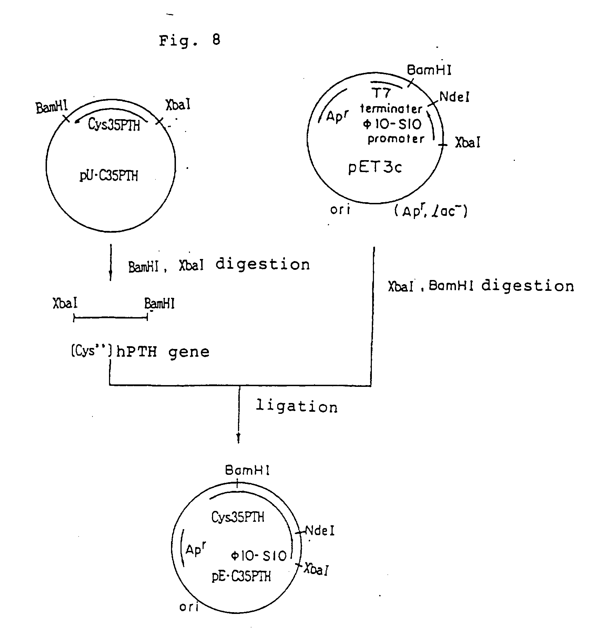

### (2) Construction of Plasmid pE-C35PTH for Expression in E. coli (Fig. 8)

pU-C35PTH obtained in (1) was digested with restriction enzymes XbaI and BamHI to obtain an about 0.3-kbp fragment coding mutein [Cys³⁵] human PTH. After purification by agarose gel electrophoresis, this fragment was ligated with T4 ligase to expression plasmid vector pET3c [F. W. Stadier et al., Methods in Enzymology 195, 60-89 (1990)] preliminarily digested with restriction enzymes XbaI and BamHI. The plasmid for expression thus obtained was named pE-C35PTH.

λ Phage DE3 [F. W. Studier et al., J. Mol. Biol., 189, 113-130 (1986)] in which an RNA polymerase gene of T7 phage was incorporated into the E. coli MM294 strain was lysogenized to prepare the E. coli MM294(DE3) strain. Using plasmid pE-C35PTH, E. coli MM294(DE3) was transformed, thereby obtaining cell MM294(DE3)/pE-C35PTH having the plasmid containing the gene coding for the mutein shown in Fig. 9.

### (3) Production of [Cys³⁵] Human PTH

(i) E. coli MM294(DE3)/pE-C35PTH was cultivated overnight at 37°C in 3 ml of LB medium containing 50 µg/ml ampicillin with shaking. To 10 ml of the same medium dispensed into a 200-ml flask, 100 µl of the resulting culture solution was added and cultivated at 37°C to a Klett of about 170. Then, IPTG was added thereto to a concentration of 0.1 mM. After cultivation was further continued for 2 hours, 1 ml of the culture solution was centrifuged at 15,000 rpm at 4°C for 5 minutes. The resulting cells were dissolved in 100 ml of a solution containing 0.5 M Tris-HCl (pH 6.8), 10% glycerol, 10% (w/v) sodium dodecyl sulfate (SDS), 0.1% (w/v) β-mercaptoethanol and bromophenol blue [U. K. Laemmli, Nature, 227, 680 (1970)]. After boiling for 3 minutes, the solution was subjected to 16% SDS-polyacrylamide gel electrophoresis (PAGE). After electrophoresis, the gel was stained with Coomassie Brilliant Blue. As a result, an intense band indicating a mobility similar to that of a standard human PTH sample was observed (refer to Fig. 14).
   Lanes 1 to 5 in Fig. 14 are as follows:
   Lane 1: Human PTH (1 µg)
   Lane 2: E. coli strain culture solution (10 µl) not carrying plasmid pE-C35PTH after induction with IPTG
   Lane 3: E. coli strain culture solution (10 µl) carrying plasmid pE-C35PTH after induction with IPTG.

   Another gel was subjected to Western blotting using an human PTH antibody. As a result, the same stained pattern as with the standard human PTH was also obtained (refer to Fig. 15). The objects of respective lanes shown in Fig. 15 are the same as with Fig. 14. From the quantitative comparison with the standard sample in gel staining, [Cys³⁵] human PTH was expressed in an amount of about 100 mg/l.
(ii) [Cys³⁵] human PTH accumulated in E. coli was purified in the following manner. Cells from 200 ml of a culture solution obtained similarly with the above-mentioned method were suspended in a buffer (5 ml) containing 8 M urea, 50 mM Tris-HCl (pH 7.5), 50 mM EDTA, 20 mM 2-mercaptoethanol (hereinafter sometimes referred to as 2-ME) and 1 mM α-toluenesulfonyl fluoride, and vigorously stirred under ice cooling for about 1 hour to disrupt the cells, followed by centrifugation at 15,000 rpm at 4°C for 20 minutes. The supernatant was recovered, and the precipitate was similarly extracted with two 3 ml portions of a buffer having the same composition twice. The extracts were combined with the supernatant, followed by two-fold dilution. The resulting solution was passed through a CM-Toyo Pearl column (10 ml) of TSK-Gel (Tosoh) equilibrated with 50 mM ammonium acetate buffer (pH 5) containing 4 M urea and 10 mM 2-mercaptoethanol (2-ME) to allow a desired product to be adsorbed. The column was washed with 50 mM ammonium acetate buffer (pH 5) containing 4 M urea and 10 mM 2-ME. About 10 ml of the buffer was required for washing. When absorption at 280 nm disappeared, the column was developed by a linear gradient of 50 ml of 50 mM ammonium acetate buffer (pH 5) containing 10 mM 2-ME-50 ml of 0.5 M ammonium acetate buffer (pH 6) (flow rate: 10 ml/hour, volume of 1 fraction: 2 ml). Fractions 35 to 43 were collected and lyophilized. These fractions were subjected to reversed phase HPLC under the following conditions:
   - Column:: YMC-Pack A-325 S-5 120A ODS (1X30 cm) (Y. M. C.)
   - Solvent:: A linear gradient of 25% to 50% acetonitrile containing 0.1% trifluoroacetic acid (for 30 minutes)
   - Flow rate:: 3 ml/minute
The peak of the desired product absorption (the retention time was 17.0 minutes) was pooled. The resulting eluate was passed through a Bio-Rad AG1X8 (acetate form) column (Bio-Rad Laboratory) and the washings were also combined therewith. Then, acetonitrile was removed by distillation, followed by lyophilization. A desired human PTH analogue was obtained in an amount of 3.6 mg as a white powder.

The following analytical results revealed that this sample was [Cys³⁵] human PTH of high purity.
a) Reversed phase HPLC showed a single sharp peak under the following condition (refer to Fig. 16).
   - Column:: YMC-Pack A-303 S-5 ODS 120A (4.6X250 mm)
   - Eluents:: Solution A [0.1% trifluoroacetic acid (hereinafter also briefly referred to as TFA)]
   Solution B (acetonitrile containing 0.1% trifluoroacetic acid)
   - Linear gradient:: 30 to 38%, B in 30 min.
b) SDS-PAGE also showed a single band of the same mobility as that of human PTH (refer to Fig. 17).
   Respective lanes shown in Fig. 17 are as follows:
   Lane 1: Molecular weight marker
   Lane 2: Human PTH
   Lane 3: [Cys³⁵] human PTH
c) Amino acid analysis (hydrolyzed with 5.7 N hydrochloric acid in the presence of thioglycolic acid in a sealed tube under reduced pressure at 110°C for 24 hours, and values in parentheses indicate theoretical values):
   Asp (10) 10.33; Thr (1) 0.91; Ser (7) 6.10; Glu (11) 11.82; Pro (3) 3.00; Gly (4) 4.44; Ala (7) 6.91; Cys (1) 1.11: Val (7) 6.60; Met (2) 2.11; Ile (1) 1.01; Leu (10) 10.83; Phe (1) 1.10; Lys (9) 9.32; His (4) 3.75; Arg (5) 5.21; Trp (1) 0.93
   (Recovery: 84.2%; for Cys, the analytical value of a hydrolysis product after oxidation with performic acid)
d) The N-terminal amino acid sequence analysis with a gas-phase sequencer Model 470A (Applied Biosystems) revealed that the sequence from Ser at the 1-position to Leu at the 15-position was correct.

Thus, the mutein having the amino acid sequence (SEQ ID:NO: 56) shown in Fig. 9 was obtained in which valine at the 35-position was substituted by cysteine.

### Example 2

### Production of Gene Coding for [Leu¹⁸] Human PTH (1-84)

### and Expression Thereof

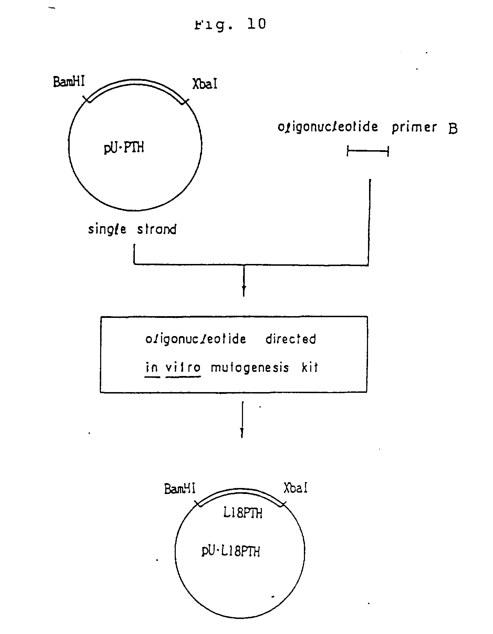

### (1) Production of Gene Coding [Leu¹⁸] Human PTH (1-84) (Fig. 10)

First, oligonucleotide primer B, ACATTTGAACTCGCTGGAGG-GTGTAGAA (SEQ ID NO: 9), was synthesized to change a codon of Met at the 18-position to a codon of Leu. Using the above-mentioned synthetic oligonucleotide (4 picomols) in which the 5' OH terminus was phosphorylated by treatment with T4 kinase and the single-stranded pU-PTH (5 µg), a plasmid was obtained into which a mutation was introduced with the site-directed mutagenesis kit (oligonucleotide-directed in vitro mutagenesis system version 2, Amersham) described in Example 2. E. coli MV1184 was normally transformed by this plasmid and seeded on a double YT medium agar plate containing 150 µg/ml ampicillin. Then, cultivation was carried out at 37°C for 15 hours to obtain many colonies. Of these, a small amount of cells were collected from 5 colonies, and cultivated on 0.3 ml of double YT medium for about 5 hours. Thirty µl of this culture solution was mixed with 30 µl of a solution containing helper pharge KO7, and the mixture was allowed to stand at 37°C for 1 hour. Then, 3 ml of double YT medium was added thereto, followed by cultivation overnight. The resulting culture solution was subjected to centrifugation to separate a supernatant from cells. A plasmid was crudely purified from the cell by the alkali method, and a single-stranded DNA existing as a phage particle was recovered from the supernatant.

The above-mentioned oligonucleotide primer B contains no recognition sites for restriction enzyme NcoI which exists in the gene coding for human PTH, said gene functioning as a template.

Accordingly, when NcoI-EcoRI is reacted with the plasmid into which a mutation is correctly introduced, the plasmid must not be cleaved at the NcoI sites existing before mutation and must be cleaved only at the EcoRI sites originally existing at multi-cloning sites in pUC118 not to produce 230-bp fragments. The plasmid obtained from the above-mentioned 5 colonies was digested with NcoI-EcoRI, and analyzed by agarose gel electrophoresis. As a result, fragments of correct size were observed in 2 clones.

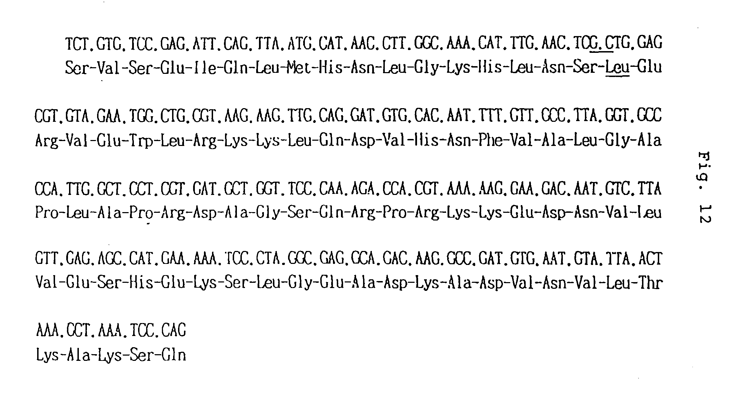

Further, using the single-stranded plasmids of these two clones as templates, the nucleotide sequence was analyzed. As a result, the introduction of the desired mutation was confirmed (Fig. 12) (SEQ ID:NO: 57).

The thus-obtained plasmid containing the gene (Fig. 9) coding for [Leu¹⁸] human PTH was named pU-L18PTH (Fig. 10).

### (2) Construction of Plasmid pE-L18PTH for Expression in E. coli

pU-L18PTH obtained in (1) was digested with restriction enzymes XbaI and BamHI to obtain an about 0.3-kbp fragment coding for mutein [Leu¹⁸] human PTH. After purification by agarose gel electrophoresis, this fragment was ligated with T4 ligase to expression plasmid vector pET3c [F. W. Stadier et al., Methods in Enzymology 195, 60-89 (1990)] preliminarily digested with restriction enzymes XbaI and BamHI. The plasmid for expression thus obtained was named pE-L18PTH.

λ Pharge DE3 [F. W. Studier et al., J. Mol. Biol., 189, 113-130 (1986)] in which an RNA polymerase gene of T7 phage was incorporated into the E. coli MM294 line was lysogenized to prepare the E. coli MM294(DE3) line.

Using plasmid pE-L18PTH, E. coli MM294(DE3) was transformed, thereby obtaining cell MM294(DE3)/pE-L18PTH having the plasmid containing the gene coding for the mutein shown in Fig. 12.

### (3) Production of Mutein [Leu¹⁸] Human PTH

(i) Cell MM294(DE3)/pE-L18PTH mentioned above was cultivated overnight in 3 ml of LB medium containing 35 µg/ml ampicillin with shaking. To 50 ml of LB medium (containing 35 µg/ml ampicillin), 2.5 µl of the resulting culture solution was added and cultivated at 37°C for 2 hours. When a Klett of about 170 is attained, IPTG was added thereto to a concentration of 0.1 mM, followed by further cultivation for 4 hours. Parts of the culture solution prior to addition of IPTG and the culture solution cultivated for 3 hours after addition thereof was subjected to centrifugation to collect cells. The resulting cells were analyzed by sodium dodecyl sulfate-polyacrylamide gel electrophoresis (SDS-PAGE) under reduced conditions. The results revealed that the expression of mutein [Leu¹⁸] human PTH was induced by addition of IPTG (Fig. 18).

Respective lanes shown in Fig. 18 are as follows:
Lane 1: Molecular weight marker
Lane 2: E. coli strain culture solution (10 µl) carrying plasmid pE-L18PTH after addition of IPTG
Lane 3: E. coli strain culture solution (10 µl) carrying plasmid pE-L18PTH without addition of IPTG
Lane 4: Human PTH expression strain culture solution (10 µl) after addition of IPTG
Lane 5: Human PTH expression strain culture solution (10 µl) without addition of IPTG.

The same transformant was cultivated in the same manner as with Example 1 (3), and a desired protein accumulated in the cells was similarly extracted and purified to give 5 mg of a pure product.

The following analytical results revealed that this sample was [Leu¹⁸] human PTH of high purity.
a) Reversed phase HPLC showed a single sharp peak under following conditions (refer to Fig. 19).
   - Column:: YMC-Pack R-ODS-5 S-5 120A (4.6X250 mm)
   - Eluents:: Solution A (0.1% TFA)
   Solution B (acetonitrile containing 0.1% TFA)
   - Elution conditions:: Linear gradient of 0 minute (23% acetonitrile containing 0.1% TFA) → 30 minutes (38% acetonitrile containing 0.1% TFA; flow rate: 1 ml/minute)
b) SDS-PAGE also showed a single band of the same mobility as that of human PTH.
c) Amino acid analysis (hydrolyzed with 5.7 N hydrochloric acid in the presence of thioglycollic acid in a sealed tube under reduced pressure at 110°C for 24 hours, and values in parentheses indicate theoretical values):
   Asp (10) 11.27; Thr (1) 0.97; Ser (7) 6.64; Glu (11) 12.0; Pro (3) 3.11; Gly (4) 3.86; Ala (7) 7.00; Val (8) 7.12; Met (1) 0.98; Ile (1) 0.98; Leu (11) 11.7; Phe (1) 1.02; Lys (9) 9.81; His (4) 3.75; Arg (5) 5.21; Trp (1) 0.93
   (Recovery: 83%)
d) The N-terminal amino acid sequence analysis with a gas-phase sequencer Model 470A (Applied Biosystems) revealed the sequence from Ser at the 1-position to Arg at the 20-position.

Thus, the mutein having the amino acid sequence (SEQ ID:NO: 57) shown in Fig. 12 was obtained in which methionine at the 18-position was substituted by leucine.

### Example 3

### Production of Gene Coding for [Leu⁸] Human PTH (1-84) and Expression Thereof

### (1) Production of Gene Coding for [Leu⁸] Human PTH (1-84)

First, oligonucleotide primer TCCGAGATTCAGCTGCTGCATAA-CCTT (SEQ ID NO: 6) was synthesized to change a codon of Met at the 8-position to a codon of Leu. Using the above-mentioned synthetic oligonucleotide (4 picomols) in which the 5' OH terminus was phosphorylated by treatment with T4 kinase and the single-stranded pU-PTH (5 µg), a plasmid was obtained into which a mutation was introduced with the site-directed mutagenesis kit (oligonucleotide-directed in vitro mutagenesis system version 2, Amersham) described in Example 2. E. coli MV1184 was transformed by this plasmid and seeded on a double YT medium agar plate containing 150 µg/ml ampicillin. Then, cultivation was carried out at 37°C for 15 hours to obtain many colonies. Of these, a small amount of cells were collected from 5 colonies, and cultivated in 0.3 ml of double YT medium for about 5 hours. Thirty µl of this culture solution was mixed with 30 µl of a solution containing helper phage KO7, and the mixture was allowed to stand at 37°C for 1 hour. Then, 3 ml of double YT medium was added thereto, followed by cultivation overnight. The resulting culture solution was subjected to centrifugation to separate a supernatant from cells. A plasmid was crudely purified from the cell by the alkali method, and a single-stranded DNA existing as a phage particle was recovered from the supernatant.

A recognition site for restriction enzyme PvuII which does not exist in the gene coding for human PTH is inserted into the above-mentioned oligonucleotide primer, said gene functioning as a template.

Accordingly, the plasmid into which a mutation is correctly introduced can be selected from the patterns of agarose gel electrophoresis after digestion with PvuII. Here, 3 clones indicated a correct digestion pattern.

Further, using the single-stranded plasmids of these two clones as templates, the nucleotide sequence was analyzed. As a result, the introduction of the desired mutation was confirmed. The thus-obtained plasmid containing the gene coding for [Leu⁸] human PTH was named pU-L8PTH.

### (2) Construction of Plasmid pE-L8PTH for Expression in E. coli

pU-L8PTH obtained in (1) was digested with restriction enzymes XbaI and BamHI to obtain an about 0.3- kbp fragment coding for mutein [Leu⁸] human PTH. After purification by agarose gel electrophoresis, this fragment was ligated with T4 ligase to expression plasmid vector pET3c [F. W. Stadier et al., Methods in Enzymology 195, 60-89 (1990)] preliminarily digested with restriction enzymes XbaI and BamHI. The plasmid for expression thus obtained was named pE-L8PTH.

λ Phage DE3 [F. W. Studier et al., J. Mol. Biol., 189, 113-130 (1986)] in which an RNA polymerase gene of T7 phage was incorporated into the E. coli MM294 strain was lysogenized to prepare the E. coli MM294(DE3) strain.

Using plasmid pE-L8PTH, E. coli MM294(DE3) was transformed, thereby obtaining cell MM294(DE3)/pE-L8PTH having the plasmid containing the gene coding for the mutein in which methionine at the 8-position was substituted by leucine.

### (3) Production of Mutein [Leu⁸] Human PTH

Cell MM294(DE3)/pE-L8PTH mentioned above was cultivated overnight in 3 ml of LB medium containing 35 µg/ml ampicillin with shaking. To 50 ml of LB medium (containing 35 µg/ml ampicillin), 2.5 µl of the resulting culture solution was added and cultivated at 37°C for 2 hours. When a Klett of about 170 is attained, IPTG was added thereto to a concentration of 0.1 mM, followed by further cultivation for 4 hours. Parts of the culture solution prior to addition of IPTG and the culture solution cultivated for 3 hours after addition thereof was subjected to centrifugation to collect cells. The resulting cells were analyzed by sodium dodecyl sulfate-polyacrylamide gel electrophoresis (SDS-PAGE) under reduced conditions. The results revealed that the expression of mutein [Leu⁸] human PTH was induced by addition of IPTG.

The same transformant was cultivated in the same manner as with Example 1 (3), and a desired protein accumulated in the cells was similarly extracted and purified to give 4.7 mg of a pure product.

The following analytical results revealed that this sample was [Leu⁸] human PTH of high purity.
a) Reversed phase HPLC showed a single sharp peak (refer to Fig. 20) under following conditions.
   - Column:: YMC-Pack R-ODS-5 S-5 120A (4.6X250 mm)
   - Eluents:: Solution A (0.1% TFA)
   Solution B (acetonitrile containing 0.1% TFA)
   - Elution conditions:: Linear gradient of 0 minute (25% acetonitrile containing 0.1% TFA) → 30 minutes (40% acetonitrile containing 0.1% TFA; flow rate: 1 ml/minute)
b) SDS-PAGE also showed a single band of the same mobility as that of human PTH.
c) Amino acid analysis (hydrolyzed with 5.7 N hydrochloric acid in the presence of thioglycolic acid in a sealed tube under reduced pressure at 110°C for 24 hours, and values in parentheses indicate theoretical values):
   Asp (10) 10.09; Thr (1) 0.89; Ser (7) 6.45; Glu (11) 11.14; Pro (3) 3.08; Gly (4) 4.03; Ala (7) 7.00; Val (8) 7.57; Met (1) 1.05; Ile (1) 1.01; Leu (11) 11.32; Phe (1) 0.98; Lys (9) 8.62; His (4) 3.72; Arg (5) 4.80; Trp (1) 0.64
   (Recovery: 79.2%)
d) The N-terminal amino acid sequence analysis with a gas-phase sequencer Model 470A (Applied Biosystems) revealed the sequence from Ser at the 1-position to Leu at the 15-position.

Thus, the mutein having the amino acid sequence (sequence No. 58) shown in Fig. 21 was obtained in which methionine at the 8-position was substituted by leucine.

### Example 4

### Production of [Leu⁸] Human PTH (7-84) Gene and Expression Thereof

### (1) Construction of Expression Plasmid for [Leu⁸] Human PTH (7-84) and Production of Transformant (Fig. 22)

(i) As a plasmid for expression, pE-PTH (European Patent Application No. 483509) in which the N-terminus of the human PTH gene was modified was used. In 15 µl of a reaction solution [10 mM Tris-HCl (pH 7.5), 10 mM MgCl₂, 1 mM DTT, 80 mM NaCl and 15 units of restriction enzyme NcoI (Takara Shuzo)], 2 µg of pE-PTH DNA was reacted at 37°C for 2 hours. Then, NaCl was added to a concentration of 150 mM, and 20 units of NdeI (New England Biolabs) was further added thereto, followed by reaction at 37°C for 2 hours. Subsequently, the reaction product was treated at 65°C for 20 minutes to inactivate the enzyme, and then, diluted 10 times with water. On the other hand, a gene coding the N-terminal portion as well as a complemental strand was synthesized using a DNA synthesizer 380A (Applied Biosystems), said gene consisting of an initiation codon ATG corresponding to the NdeI binding site, a codon for Leu⁷ subsequent thereto, a codon of Leu substituted for Met⁸ and codons of His⁹ to Ser¹⁷ corresponding to the NcoI binding site. The 5'-termini of these two fragments were each phosphorylated. Referring to Fig. 22, the mark 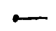 indicates that a 5'-terminal hydroxyl group is phosphorylated.

5'-TATGTTACTCCATAACCTTGGCAAACATTTGAACTC-3' (SEQ ID NO:
59)

5′-CATGGAGTTCAAATGTTTGCCAAGGTTATGGAGTAACA-3′ (SEQ ID
NO: 60)

Both of them were mixed with each other, and the mixture was reacted with T4 DNA ligase (New England Biolabo) in the presence of 50 mM Tris-HCl (pH 7.6), 10 mM MgCl₂, 10 mM DTT, 1 mM spermidine, 0.1 mg/ml BSA and 1 mM ATP at 14°C for 16 hours to ligate the DNAs to each other, followed by transformation of the E. coli JM109 strain in the same manner as above. Then, this E. coli was seeded onto LB medium containing 50 µg/ml ampicillin, and cultivated at 37°C for 1 day. The resulting ampicillin-resistant colony was selected. A plasmid DNA of the transformed strain was further digested with restriction enzymes NdeI-BamHI. The transformed strain correctly containing the human PTH gene was selected by its pattern of polyacrylamide electrophoresis. The plasmid for expression thus obtained was named pE-L8PTH (7-84), and the transformed strain was named E. coli JM109/pE-L8PTH (7-84).

(ii) A plasmid DNA was isolated from JM109/pE-L8PTH (7-84) obtained in (i). Using this plasmid DNA, E. coli MM294(DE3) was transformed similarly with Reference Example 7 (3) (ii) to obtain E. coli MM294(DE3)/pE-L8PTH (7-84).

### (2) Production of [Leu⁸] Human PTH (7-84)

E. coli MM294(DE3)/pE-L8PTH (7-84) obtained in (1) was cultivated similarly with Example 1 (3), and a desired protein (Fig. 23) accumulated in the cells was extracted and purified to give 5.3 mg of a product. The amino acid analysis and the N-terminal amino acid sequence analysis revealed that Met is further added to the N-terminal Leu of the above protein, namely that the protein is [Met⁶, Leu⁸] human PTH (6-84).

The following analytical results revealed that this sample was [Leu⁸] human PTH (7-84) of high purity (with the proviso that a Met residue was added to the N-terminus).a) Reversed phase HPLC showed a single sharp peak under following conditions (refer to Fig. 24).
- Column:: YMC-Pack R-ODS-5 S-5 120A (4.6x250 mm)
- Eluents:: Solution A (0.1% TFA)
Solution B (acetonitrile containing 0.1% TFA)
- Elution conditions:: Linear gradient of 0 minute (23% acetonitrile containing 0.1% TFA) → 30 minutes (38% acetonitrile containing 0.1% TFA; flow rate: 1 ml/minute)

b) SDS-PAGE also showed a single band.
c) Amino acid analysis (hydrolyzed with 5.7 N hydrochloric acid in the presence of thioglycolic acid in a sealed tube under reduced pressure at 110°C for 24 hours, and values in parentheses indicate theoretical values):
   Asp (10) 10.1; Thr (1) 0.91; Ser (5) 4.49; Glu (9) 9.46; Pro (3) 2.96; Gly (4) 3.67; Ala (7) 7.00; Val (7) 6.35; Met (1) 1.94; Leu (11) 11.41; Phe (1) 1.07; Lys (9) 8.62; His (4) 3.61; Arg (5) 4.89; Trp (1) 0.91
   (Recovery: 79.7%)
d) The N-terminal amino acid sequence analysis with a gas-phase sequencer Model 470A (Applied Biosystems) revealed that the sequence from Ser at the 1-position and Leu at the 2-position to the twentieth residue was correct.

Thus, the mutein having the amino acid sequence (sequence No. 61) shown in Fig. 25, human PTH (7-84) in which methionine at the 8-position was substituted by leucine, was obtained.

### Example 5

### Production of [Leu^{8,18}] Human PTH (1-84) and Expression

### Thereof

### (1) Production of Gene Coding [Leu^{8,18}] Human PTH (1-84) (Fig. 26)

Using plasmid pE-L8PTH for expression of [Leu⁸] human PTH obtained in Example3 , single-stranded plasmid pU-L8PTH containing a [Leu⁸] human PTH gene for conducting site-directed mutagenesis was constructed by a procedure similar to that for obtaining pU-PTH described in Reference Example 3. Using this DNA, plasmid pU-L8,18PTH was obtained which contains a gene coding for [Leu^{8,18}] human PTH in which the 18-position Met codon was mutated to a Leu codon by a method similar to that described in Example 2.

### (2) Construction of Plasmid pE-L8,18PTH for Expression in E. coli (Fig. 27)

Using pU-L8,18PTH obtained in (1), plasmid pE-L8,18PTH for expression was obtained in the same manner as with Example 3 (2). Then, E. coli MM294(DE3) was transformed using this plasmid, thereby obtaining cell MM294(DE3)/pE-L8,18PTH which has a plasmid containing a gene coding for the mutein shown in Fig. 28 (SEQ ID NO: 62).

### (3) Production of [Leu^{8,18}] Human PTH

Transformant MM294(DE3)/pE-L^{8,18}PTH obtained in (2) was cultivated in the same manner as with Example 1 (3), and a desired protein accumulated in the cells was similarly extracted and purified to give 5 mg of pure [Leu^{8,18}] human PTH (1-84).

The following analytical results revealed that this sample was [Leu^{8,18}] human PTH of high purity.
a) Reverse phase HPLC showed a single sharp peak (refer to Fig. 29) under following conditions.
   - Column:: YMC-Pack R-ODS-5 S-5 120A (4.6X250 mm)
   - Eluents:: Solution A (0.1% TFA)
   Solution B (acetonitrile containing 0.1% TFA)
   - Elution conditions:: Linear gradient of 0 minute (25% acetonitrile containing 0.1% TFA) → 30 minutes (40% acetonitrile containing 0.1% TFA; flow rate: 1 ml/minute)
b) SDS-PAGE also showed a single band of the same mobility as that of human PTH.
c) Amino acid analysis (hydrolyzed with 5.7 N hydrochloric acid in the presence of thioglycollic acid in a sealed tube under reduced pressure at 110°C for 24 hours, and values in parentheses indicate theoretical values):
   Asp (10) 10.38; Thr (1) 0.88; Ser (7) 6.37; Glu (11) 11.82; Pro (3) 2.88; Gly (4) 3.79; Ala (7) 7.00; Val (8) 7.47; Ile (1) 0.98; Leu (12) 13.00; Phe (1) 1.10; Lys (9) 8.93; His (4) 3.74; Arg (5) 5.09; Trp (1) 0.96
   (Recovery: 80%)
d) The N-terminal amino acid sequence analysis with a gas-phase sequencer Model 470A (Applied Biosystems) revealed that the sequence from Ser at the 1-position to Arg at the 20-position was correct.

Thus, the mutein having the amino acid sequence (SEQ ID:NO: 62) shown in Fig. 28 was obtained in which methionine residues at the 8- and the 18-positions were substituted by leucine.

### Experimental Example

The biological activity of human PTH muteins obtained in Examples 1 to 5 and natural type human PTH were evaluated by a modification of the method reported by Shigeno et al.[The Journal of Biological Chemistry 263. 18369-18377 (1988)]. A culture solution (Hank's solution containing 20 mM N-2-hydroxyethylpiperazine-N'-2-ethanesulfonic acid (HEPES), 0.1% bovine serum albumin (BSA) and 0.5 mM isobutylmethylxanthine) containing 0.01, 0.1, 1, 10 or 100 nM analogue was added in an amount of 100 µl to mouse cranial bone-derived osteoblast-like cell strain MC3T3-EI cultivated on a 96-well multiplate (Nunclon, Nunc), followed by reaction at room temperature for 30 minutes. After addition of 100 µl of 0.2 N hydrochloric acid, the mixture was immersed in boiling water for 2.5 minutes, and cyclic adenosine monophosphate (cAMP) produced by a human PTH receptor was extracted from the cells. The total cAMP in the culture solution and the cells was assayed using a commercial radioimmunoassay kit (cyclic AMP [¹²⁵I] kit "Du Pont-Daiichi", Daiichi Kagaku Yakuhin). An increase in cAMP production depending on the concentration of the human PTH mutein added was always observed. The specific activity of the muteins on the natural type human PTH is as follows:

| Human PTH | Specific Activity |
|---|---|
| Human PTH | 1.0 |
| [Leu⁸] human PTH | 1.0 |
| [Leu¹⁸] human PTH | 0.2 |
| [Leu^{8,18}] human PTH | 0.3 |
| [Cys³⁵] human PTH | 0.6 |

## Claims (Claims for the following Contracting State(s): AT, BE, CH, DE, DK, FR, GB, GR, IE, IT, LI, LU, NL, PT, SE)

1. A human parathyroid hormone mutein which comprises at least one modification selected from the group consisting of (1) deletion of 3 to 6 amino acid residues on the N-terminal side in the amino acid sequence of human parathyroid hormones, (2) substitution of another lipophilic amino acid residue for at least one methionine residue in said amino acid sequence, and (3) substitution of a cysteine residue for one amino acid residue within the region of amino acid residue Nos. 34 to 47 in said amino acid sequence, provided that a deletion according to (1) is accompanied by a substitution according to (2) and/or (3).

2. The human parathyroid hormone mutein claimed in claim 1, wherein 3 to 6 N-terminal amino acid residues are deleted and at least one methionine residue is substituted by another lipophilic amino acid residue.

3. The human parathyroid hormone mutein claimed in claim 1, wherein 3 to 6 N-terminal amino acid residues are deleted and one amino acid residue within the region of amino acid residue Nos. 34 to 47 is substituted by a cysteine residue.

4. The human parathyroid hormone mutein claimed in claim 1, wherein at least one methionine residue is substituted by another lipophilic amino acid residue and one amino acid residue within the region of amino acid residue Nos. 34 to 47 is substituted by a cysteine residue.

5. The human parathyroid hormone mutein claimed in claim 4, wherein 3 to 6 N-terminal amino acid residues are further deleted.

6. The human parathyroid hormone mutein claimed in claim 1, wherein one amino acid residue within the region of amino acid residue Nos. 34 to 47 is substituted by a cysteine residue.

7. The human parathyroid hormone mutein claimed in claim 1, which is [Cys³⁵] human parathyroid hormone (1-84).

8. A recombinant DNA having a nucleotide sequence coding for the human parathyroid hormone mutein claimed in claim 1.

9. A vector containing the recombinant DNA claimed in claim 8.

10. A vector in which the recombinant DNA claimed in claim 8 is inserted into a region controlled by an Escherichia coli T7 promoter.

11. A transformant which is transformed by the recombinant DNA claimed in claim 8.

12. A process for producing a human parathyroid hormone mutein which comprises cultivating the transformant claimed in claim 11 in a culture medium.

## Claims (Claims for the following Contracting State(s): ES)

1. A process for producing a human parathyroid hormone mutein which comprises at least one modification selected from the group consisting of (1) deletion of 3 to 6 amino acid residues on the N-terminal side in the amino acid sequence of human parathyroid hormones, (2) substitution of another lipophilic amino acid residue for at least one methionine residue in said amino acid sequence, and (3) substitution of a cysteine residue for one amino acid residue within the region of amino acid residue Nos. 34 to 47 in said amino acid sequence, provided that a deletion according to (1) is accompanied by a substitution according to (2) and/or (3), which process comprises cultivating a transformant in a culture medium, wherein the transformant is transformed by a recombinant DNA having a nucleotide sequence coding for the human parathyroid hormone mutein as defined above.

2. The process of claim 1, wherein in the human parathyroid hormone mutein 3 to 6 N-terminal amino acid residues are deleted and at least one methionine residue is substituted by another lipophilic amino acid residue.

3. The process of claim 1, wherein in the human parathyroid hormone mutein 3 to 6 N-terminal amino acid residues are deleted and one amino acid residue within the region of amino acid residue Nos. 34 to 47 is substituted by a cysteine residue.

4. The process of claim 1, wherein in the human parathyroid hormone mutein at least one methionine residue is substituted by another lipophilic amino acid residue and one amino acid residue within the region of amino acid residue Nos. 34 to 47 is substituted by a cysteine residue.

5. The process of claim 4, wherein 3 to 6 N-terminal amino acid residues are further deleted in the human parathyroid hormone mutein.

6. The process of claim 1, wherein in the human parathyroid hormone mutein one amino acid residue within the region of amino acid residue Nos. 34 to 47 is substituted by a cysteine residue.

7. The process of claim 1, wherein the produced human parathyroid hormone mutein is [Cys³⁵] human parathyroid hormone (1-84).

8. A process for producing a recombinant DNA having a nucleotide sequence coding for the human parathyroid hormone mutein as defined in claim 1, which process comprises site-directed mutagenesis.

9. A process for producing a vector containing the recombinant DNA defined in claim 8 which process comprises ligating the recombinant DNA in the vector.

10. The process of claim 9 in which the recombinant DNA defined in claim 8 is inserted into a region controlled by an Escherichia coli T7 promoter.

11. A process for producing a transformant which is transformed by the recombinant DNA defined in claim 8 which process comprises transforming a host cell with the vector produced according to claim 9.

## Revendications (Revendications pour l'(les) Etat(s) contractant(s) suivant(s): AT, BE, CH, DE, DK, FR, GB, GR, IE, IT, LI, LU, NL, PT, SE)

1. Mutéine d'hormone parathyroïdienne humaine comprenant au moins une modification choisie dans le groupe formé par
(1) une délétion de 3 à 6 résidus d'acides aminés de l'extrémité N-terminale de la séquence d'acides aminés d'hormones parathyroïdiennes humaines,
(2) une substitution d'au moins un résidu méthionine de ladite séquence d'acides aminés par un autre acide aminé lipophile, et
(3) une substitution d'un résidu d'acide aminé dans la région des résidus d'acides aminés n° 34 à 47 de ladite séquence par un résidu cystéine,
à condition que la délétion conforme à (1) soit accompagnée d'une substitution conforme à (2) et/ou (3).

2. Mutéine d'hormone parathyroïdienne humaine conforme à la revendication 1 dans laquelle de 3 à 6 résidus d'acides aminés de l'extrémité N-terminale ont été éliminés et au moins un résidu méthionine a été remplacé par un autre acide aminé lipophile.

3. Mutéine d'hormone parathyroïdienne humaine conforme à la revendication 1 dans laquelle de 3 à 6 résidus d'acides aminés de l'extrémité N-terminale ont été éliminés et un résidu d'acide aminé dans la région des résidus d'acides aminés n° 34 à 47 a été remplacé par un résidu cystéine.

4. Mutéine d'hormone parathyroidienne humaine conforme à la revendication 1 dans laquelle au moins un résidu méthionine a été remplacé par un autre acide aminé lipophile et un résidu d'acide aminé dans la région des acides aminés n° 34 à 47 a été remplacé par un résidu cystéine.

5. Mutéine d'hormone parathyroïdienne humaine conforme à la revendication 4 dans laquelle on a également éliminé de 3 à 6 résidus d'acides aminés de l'extrémité N-terminale.

6. Mutéine d'hormone parathyroïdienne humaine conforme à la revendication 1 dans laquelle un résidu d'acide aminé de la région des acides aminés n° 34 à 47 a été remplacé par un résidu cystéine.

7. Mutéine d'hormone parathyroïdienne humaine conforme à la revendication 1 qui est la [³⁵Cys] hormone parathyroïdienne humaine (1-84).

8. ADN recombiné ayant une séquence nucléotidique codant la mutéine d'hormone parathyroïdienne humaine conforme à la revendication 1.

9. Vecteur renfermant l'ADN recombiné conforme à la revendication 8.

10. Vecteur dans lequel l'ADN recombiné conforme à la revendication 8 est inséré dans une région qui est sous le contrôle du promoteur de T7 d'Escherichia coli.

11. Cellule transformée par l'ADN recombiné conforme à la revendication 8.

12. Procédé de préparation d'une mutéinc d'hormone parathyroïdienne humaine comprenant la culture des cellules transformées conformes à la revendication 11 dans un milieu de culture.

## Revendications (Revendications pour l'(les) Etat(s) contractant(s) suivant(s): ES)

1. Procédé de préparation d'une mutéine d'hormone parathyroïdienne humaine comprenant au moins une modification choisie dans le groupe formé par
(1) une délétion de 3 à 6 résidus d'acides aminés de l'extrémité N-terminale de la séquence d'acides aminés d'hormones parathyroïdiennes humaines,
(2) une substitution d'au moins un résidu méthionine de ladite séquence d'acides aminés par un autre acide aminé lipophile, et
(3) une substitution d'un résidu d'acide aminé dans la région des résidus d'acides aminés n° 34 à 47 de ladite séquence par un résidu cystéine,
à condition que la délétion conforme à (1) soit accompagnée d'une substitution conforme à (2) et/ou (3), lequel procédé comprend la culture de cellules transformées dans un milieu de culture, les cellules transformées ayant été transformées par de l'ADN recombiné ayant une séquence nucléotidique codant la mutéine d'hormonc parathyroïdienne humaine définie ci-dessus.

2. Procédé conforme à la revendication 1 dans lequel, dans la mutéine d'hormone parathyroïdienne humaine, de 3 à 6 résidus d'acides aminés de l'extrémité N-terminale ont été éliminés et au moins un résidu méthionine a été remplacé par un autre acide aminé lipophile.

3. Procédé conforme à la revendication 1 dans lequel, dans la mutéine d'hormone parathyroïdienne humaine, de 3 à 6 résidus d'acides aminés de l'extrémité N-terminale ont été éliminés et un résidu d'acide aminé dans la région des résidus d'acides aminés n° 34 à 47 a été remplacé par un résidu cystéine.

4. Procédé conforme à la revendication 1 dans lequel, dans la mutéine d'hormone parathyroïdienne humaine, au moins un résidu méthionine a été remplacé par un autre acide aminé lipophile et un résidu d'acide aminé dans la région des acides aminés n° 34 à 47 a été remplacé par un résidu cystéine.

5. Procédé conforme à la revendication 4 dans lequel, dans la mutéine d'hormone parathyroïdienne humaine on a également éliminé de 3 à 6 résidus d'acides aminés de l'extrémité N-terminale.

6. Procédé conforme à la revendication 1 dans lequel, dans la mutéine d'hormone parathyroïdienne humaine, un résidu d'acide aminé de la région des acides aminés n° 34 à 47 a été rcmplacé par un résidu cystéine.

7. Procédé conforme à la revendication 1 dans lequel la mutéine d'hormone parathyroïdienne humaine préparée est la [³⁵Cys] hormone parathyroïdienne humaine (1-84).

8. Procédé de préparation d'ADN recombiné ayant une séquence nucléotidiquc codant la mutéine d'hormone- parathyroidiennc humaine conforme à la revendication 1 lequel procédé comprend de la mutagénèse dirigée.

9. Procédé de préparation d'un vecteur renfermant l'ADN recombiné conforme à la revendication 8, lequel procédé comprend l'insertion de l'ADN recombiné dans le vecteur à l'aide d'une ligase.

10. Procédé conforme à la revendication 9 dans lequel l'ADN recombiné conforme à la revendication 8 est inséré dans une région qui est sous le contrôle du promoteur de T7 d'Escherichia coli.

11. Procédé de préparation de cellules transformées par l'ADN recombiné conforme à la revendication 8, lequel procédé comprend la transformation de cellules hôtes par le vecteur préparé conformément à la revendication 9.

## Patentansprüche (Patentansprüche für folgende(n) Vertragsstaat(en): AT, BE, CH, DE, DK, FR, GB, GR, IE, IT, LI, LU, NL, PT, SE)

1. Menschliches Nebenschilddrüsenhormon-Mutein, das wenigstens eine Modifikation umfaßt, die aus der Gruppe ausgewählt ist, welche aus einer (1) Deletion von 3 bis 6 Aminosäureresten an der N-terminalen Seite der Aminosäurensequenz menschlicher Nebenschilddrüsenhormone, (2) Substitution wenigstens eines Methioninrestes in der Aminosäurensequenz durch einen anderen lipophilen Aminosäurerest und (3) Substitution eines Aminosäurerestes innerhalb des Bereichs der Aminosäurereste Nr. 34 bis 47 in der Aminosäurensequenz durch einen Cysteinrest besteht, vorausgesetzt, daß eine Deletion gemäß (1) von einer Substitution gemäß (2) und/oder (3) begleitet wird.

2. In Anspruch 1 beanspruchtes menschliches Nebenschilddrüsenhormon-Mutein, bei dem 3 bis 6 N-terminale Aminosäurereste ausgelassen sind und wenigstens ein Methioninrest durch einen anderen lipophilen Aminosäurerest ersetzt ist.

3. In Anspruch 1 beanspruchtes menschliches Nebenschilddrüsenhormon-Mutein, bei dem 3 bis 6 N-terminale Aminosäurereste ausgelassen sind und ein Aminosäurerest innerhalb des Bereichs der Aminosäurereste Nr. 34 bis 47 durch einen Cysteinrest ersetzt ist.

4. In Anspruch 1 beanspruchtes menschliches Nebenschilddrüsenhormon-Mutein, bei dem wenigstens ein Methioninrest durch einen anderen lipophilen Aminosäurerest ersetzt ist und ein Aminosäurerest innerhalb des Bereichs der Aminosäurereste Nr. 34 bis 47 durch einen Cysteinrest ersetzt ist.

5. In Anspruch 4 beanspruchtes menschliches Nebenschilddrüsenhormon-Mutein, bei dem weitere 3 bis 6 N-terminale Aminosäurereste ausgelassen sind.

6. In Anspruch 1 beanspruchtes menschliches Nebenschilddrüsenhormon-Mutein, bei dem ein Aminosäurerest innerhalb des Bereichs der Aminosäurereste Nr. 34 bis 47 durch einen Cysteinrest ersetzt ist.

7. In Anspruch 1 beanspruchtes menschliches Nebenschilddrüsenhormon-Mutein, das menschliches [Cys³⁵] - Nebenschilddrüsenhormon (1-84) ist.

8. Rekombinante DNA mit einer für das in Anspruch 1 beanspruchte menschliche Nebenschilddrüsenhormon-Mutein kodierenden Nukleotidsequenz.

9. Vektor, der die in Anspruch 8 beanspruchte rekombinante DNA enthält.

10. Vektor, bei dem die in Anspruch 8 beanspruchte rekombinante DNA in eine durch einen *Escherichia coli*-T7-Promotor kontrollierte Region inseriert ist.

11. Transformante, die durch die in Anspruch 8 beanspruchte rekombinante DNA transformiert ist.

12. Verfahren zum Herstellen eines menschlichen Nebenschilddrüsenhormon-Muteins, welches das Kultivieren der in Anspruch 11 beanspruchten Transformante in einem Kulturmedium umfaßt.

## Patentansprüche (Patentansprüche für folgende(n) Vertragsstaat(en): ES)

1. Verfahren zum Herstellen eines menschlichen Nebenschilddrüsenhormon-Muteins, das wenigstens eine Modifikation umfaßt, die aus der Gruppe ausgewählt ist, welche aus einer (1) Deletion von 3 bis 6 Aminosäureresten an der N-terminalen Seite der Aminosäurensequenz menschlicher Nebenschilddrüsenhormone, (2) Substitution wenigstens eines Methioninrestes in der Aminosäurensequenz durch einen anderen lipophilen Aminosäurerest und (3) Substitution eines Aminosäurerestes innerhalb des Bereichs der Aminosäurereste Nr. 34 bis 47 in der Aminosäurensequenz durch einen Cysteinrest besteht, vorausgesetzt, daß eine Deletion gemäß (1) von einer Substitution gemäß (2) und/oder (3) begleitet wird, wobei das Verfahren das Kultivieren einer Transformante in einem Kulturmedium umfaßt, wobei die Transformante durch eine rekombinante DNA mit einer für das vorstehend definierte menschliche Nebenschilddrüsenhormon-Mutein kodierenden Nukleotidsequenz transformiert ist.

2. Verfahren von Anspruch 1, wobei in dem menschlichen Nebenschilddrüsenhormon-Mutein 3 bis 6 N-terminale Aminosäurereste ausgelassen sind und wenigstens ein Methioninrest durch einen anderen lipophilen Aminosäurerest ersetzt ist.

3. Verfahren von Anspruch 1, wobei in dem menschlichen Nebenschilddrüsenhormon-Mutein 3 bis 6 N-terminale Aminosäurereste ausgelassen sind und ein Aminosäurerest innerhalb des Bereichs der Aminosäurereste Nr. 34 bis 47 durch einen Cysteinrest ersetzt ist.

4. Verfahren von Anspruch 1, wobei in dem menschlichen Nebenschilddrüsenhormon-Mutein wenigstens ein Methioninrest durch einen anderen lipophilen Aminosäurerest ersetzt ist und ein Aminosäurerest innerhalb des Bereichs der Aminosäurereste Nr. 34 bis 47 durch einen Cysteinrest ersetzt ist.

5. Verfahren von Anspruch 4, wobei in dem menschlichen Nebenschilddrüsenhormon-Mutein weitere 3 bis 6 N-terminale Aminosäurereste ausgelassen sind.

6. Verfahren von Anspruch 1, wobei in dem menschlichen Nebenschilddrüsenhormon-Mutein ein Aminosäurerest innerhalb des Bereichs der Aminosäurereste Nr. 34 bis 47 durch einen Cysteinrest ersetzt ist.

7. Verfahren von Anspruch 1, wobei das hergestellte menschliche Nebenschilddrüsenhormon-Mutein menschliches [Cys³⁵]-Nebenschilddrüsenhormon (1-84) ist.

8. Verfahren zum Herstellen einer rekombinanten DNA mit einer für das in Anspruch 1 definierte menschliche Nebenschilddrüsenhormon-Mutein kodierenden Nukleotidsequenz, wobei das Verfahren eine ortsgerichtete Mutagenese umfaßt.

9. Verfahren zum Herstellen eines Vektors, der die in Anspruch 8 definierte rekombinante DNA enthält, wobei das Verfahren das Ligieren der rekombinanten DNA in dem Vektor umfaßt.

10. Verfahren von Anspruch 9, bei dem die in Anspruch 8 definierte rekombinante DNA in eine durch einen *Escherichia coli-*T7-Promotor kontrollierte Region inseriert wird.

11. Verfahren zum Herstellen einer Transformante, die durch die in Anspruch 8 definierte rekombinante DNA transformiert ist, wobei das Verfahren das Transformieren einer Wirtszelle mit dem gemäß Anspruch 9 hergestellten Vektor umfaßt.
